# EUROPEAN PATENT APPLICATION

(11) **EP 4 063 378 A1**
(43) Date of publication of application: **28.09.2022**
(21) Application number: 20889188.7
(22) Date of filing: 20.11.2020
(51) Int. Cl.: C07K 7/06, A61K 38/08, A61P 11/06

(54) **BIOLOGICAL PEPTIDE FOR TREATING LUNG DISEASES AND APPLICATION THEREOF**

(30) Priority: 21.11.2019 CN 201911151330; 21.11.2019 CN 201911150271; 19.06.2020 CN 202010568266; 19.06.2020 CN 202010568997
(71) Applicant: Shanghai Institute of Pharmaceutical Industry Co., Ltd., Shanghai, 200040 (CN); China State Institute of Pharmaceutical Industry Co,. Ltd., China (Shanghai) Pilot Free Trade Zone Pudong New Area, Shanghai 201203 (CN)
(72) Inventor: LIU, Li, Beijing Road, Jing'an District Shanghai 200040 (CN); MEI, Qibing, Beijing Road, Jing'an District Shanghai 200040 (CN); MA, Shumei, Beijing Road, Jing'an District Shanghai 200040 (CN); LI, Liang, Beijing Road, Jing'an District Shanghai 200040 (CN); LIU, Nan, Beijing Road, Jing'an District Shanghai 200040 (CN); GU, Fenghua, Beijing Road, Jing'an District Shanghai 200040 (CN); WANG, Jiahui, Beijing Road, Jing'an District Shanghai 200040 (CN); XU, Wenqi, Beijing Road, Jing'an District Shanghai 200040 (CN)
(74) Representative: Cabinet Laurent & Charras
(86) International application number: PCT/CN2020/130625
(87) International publication number: WO 2021/098854

(57) **Abstract**

Provided is a biological peptide for treating lung diseases and an application thereof. Also provided is a preparation method and an application of the polypeptide, as well as a pharmaceutical composition containing the polypeptide. The polypeptide of the present invention has many advantages, such as small molecular weight, low production cost, excellent water solubility, excellent stability, long half-life, low immunogenicity, low toxic side effects, and strong tissue penetration. Moreover, the polypeptide of the present invention has significant functions of preventing, treating, and/or alleviating lung diseases such as asthma and chronic obstructive pulmonary disease.

SEQUENCE LISTING

<110>  SHANGHAI INSTITUTE OF PHARMACEUTICAL INDUSTRY CO., LTD.
       CHINA STATE INSTITUTE OF PHARMACEUTICAL INDUSTRY CO., LTD.

<120>  BIOLOGICAL PEPTIDE FOR TREATING LUNG DISEASES AND APPLICATION THEREOF

<130>  X7-B-65525 EP

<140>  EP20889188.7
<141>  2020-11-20

<150>  CN201911151330.X
<151>  2019-11-21

<150>  CN201911150271.4
<151>  2019-11-21

<150>  CN202010568266.1
<151>  2020-06-19

<150>  CN202010568997.6
<151>  2020-06-19

<160>  2

<170>  PatentIn version 3.5

<210>  1
<211>  7
<212>  PRT
<213>  Artificial Sequence

<220>
<223>  SP

<400>  1

Gly Gln Thr Tyr Thr Ser Gly
1               5

<210>  2
<211>  7
<212>  PRT
<213>  Artificial Sequence

<220>
<223>  SIPI-D00

<400>  2

Gly Ser Thr Tyr Thr Gln Gly
1               5

## Description

### Technical Field

The invention relates to the field of biomedicine, and in particular relates to a biological peptide for treating lung diseases and application thereof.

### Background

Polypeptides are a class of substances that are the same as proteins in terms of amino acid composition and connection method, but different from proteins in some properties, such as simpler spatial structure, low or no immunogenicity, and strong physiological activity, etc. However, the inherent characteristics of polypeptide substances, such as low oral utilization rate, high enzymatic degradability, and extremely short half-life, limit their application in drug development. An important reason for the instability of polypeptide drugs is the special molecular structure of polypeptides.

Pulmonary diseases such as asthma and chronic obstructive pulmonary disease are intractable lung diseases, and it is difficult for existing clinical drugs to effectively treat asthma and chronic obstructive pulmonary disease. With the development of biomedical technology, many peptides have been developed for asthma and chronic obstructive pulmonary disease. However, these peptides have poor stability, short half-life *in vivo,* and are difficult to be used in effective treatment. Therefore, they are limited as therapeutic drugs.

Therefore, there is an urgent need in the art to develop a drug with strong stability, long effect *in vivo* and high curative effect, for the effective treatment of pulmonary diseases such as asthma and chronic obstructive pulmonary disease.

### SUMMARY OF THE INVENTION

The present invention improves the metabolic stability of the 7-peptide through structural changes and modifications (such as glycosylation modification), making it possible to develop and apply it as a drug for treating pulmonary diseases such as asthma and chronic obstructive pulmonary disease.

In the first aspect of the present invention, it provides a polypeptide as shown in Formula I or a pharmaceutically acceptable salt thereof, the polypeptide or the pharmaceutically acceptable salt thereof has the activity of preventing, treating and/or alleviating asthma;

XO-X1-X2-X3-X4-X5-X6-X7-X8 (I)

wherein,
X0 and X8 are each independently none, or a peptide of 1-3 amino acids;
X1 is an amino acid selected from the group consisting of Gly, Pro, Ala;
X2 is an amino acid selected from the group consisting of Ser, Thr;
X3 is an amino acid selected from the group consisting of Thr, Ser;
X4 is an amino acid selected from the group consisting of Tyr, Trp, Phe, Thr, Ser;
X5 is an amino acid selected from the group consisting of Thr, Ser;
X6 is an amino acid selected from the group consisting of Gln, Asn;
X7 is an amino acid selected from the group consisting of Gly, Pro, Ala.

In another preferred embodiment, one or more amino acids of X0-X8 are D-type amino acids.

In another preferred embodiment, the amino acids in X0-X8 are each independently a D-type amino acid or an L-type amino acid.

In another preferred embodiment, the amino acids in X0-X8 are each independently a D-type amino acid.

In another preferred embodiment, the polypeptide shown in Formula I or the pharmaceutically acceptable salt thereof comprises one or more features selected from the group consisting of:
X0 and X8 are each independently none;
X1 is D-Gly;
X2 is D-Se;
X3 is D-Thr;
X4 is D-Tyr;
X5 is D-Th;
X6 is D-Gln; and/or
X7 is D-Gly.

In another preferred embodiment, 1-2, preferably 3-5, more preferably 6-9 amino acids in X0-X8 are D-type amino acids.

In another preferred embodiment, the total length of the polypeptide is ≤20 amino acid residues, preferably ≤13 amino acid residues, preferably ≤12 amino acid residues, and preferably <10 amino acid residues amino acid residues, more preferably ≤8 amino acid residues, more preferably ≤7 amino acid residues, and most preferably is 7 amino acid residues.

In another preferred embodiment, both X0 and X8 are none or peptides with 1-3 amino acids.

In another preferred embodiment, both X0 and X8 are none.

In another preferred embodiment, the polypeptide is selected from thegroup consisting of:
(a) a polypeptide having the amino acid sequence shown in SEQ ID NO: 2, and the length of the polypeptide is 5-20 amino acids (preferably 8-12 amino acids);
(b) a derivative polypeptide derived from (a), which is formed by substitution, deletion or addition of 1-2 amino acid residues of amino acid sequence shown in SEQ ID NO: 2, and has the function of preventing, treating and/or alleviating asthma.

In another preferred embodiment, the derivative polypeptide retains ≥70%, preferably 80%, more preferably 85%, and most preferably 90% or more than 95% of activity of the polypeptide shown in SEQ ID NO: 2 for preventing, treating and/or alleviating asthma.

In another preferred embodiment, the homology between the derivative polypeptide and SEQ ID NO: 2 is ≥80%, preferably ≥90%; more preferably ≥95%.

In another preferred embodiment, the polypeptide is formed by substitution of 1-5, preferably 1-3, more preferably 1-2 amino acids; and/or
deletion of 1-3, preferably 1-2 amino acids of the polypeptide shown in SEQ ID NO.: 2; and/or
addition of 1-3, more preferably 1-2 amino acids in the two terminals of the polypetide respectively.

In another preferred embodiment, the amino acid sequence of the polypeptide is shown in SEQ ID NO: 2.

In another preferred embodiment, the amino acid sequence of the polypeptide is shown in SEQ ID NO: 2, and all the amino acids in the amino acid sequence shown in SEQ ID NO: 2 are D-type amino acids.

In another preferred embodiment, the asthma includes: allergic asthma, non-allergic asthma, late-onset asthma, airflow-restricted asthma, obesity-type asthma, and hormone-resistant asthma.

In the second aspect of the present invention, it provides an isolated nucleic acid molecule encoding the polypeptide of the first aspect of the present invention.

In the third aspect of the present invention, it provides a pharmaceutical composition, wherein the pharmaceutical composition comprising:
(a) the polypeptide according to the first aspect of the present invention or a pharmaceutically acceptable salt thereof; and
(b) a pharmaceutically acceptable carrier or excipient.

In another preferred embodiment, the component (a) accounts for 0.1-99.9 wt %, preferably 10-99.9 wt %, more preferably 70-99.9 wt % of the total weight of the pharmaceutical composition.

In another preferred embodiment, the pharmaceutical composition is liquid, solid, or semi-solid.

In another preferred embodiment, the dosage form of the pharmaceutical composition is an oral dosage form, an injection, a spray, an aerosol or an external pharmaceutical dosage form.

In another preferred embodiment, the dosage form of the pharmaceutical composition includes tablets, granules, capsules, oral liquids, or injections.

In another preferred embodiment, the pharmaceutical composition is a liquid composition.

In another preferred embodiment, the pharmaceutical composition is an oral preparation.

In another preferred embodiment, the dosage form of the pharmaceutical composition is a subcutaneous injection or an intramuscular injection.

In another preferred embodiment, the pharmaceutically acceptable carrier is selected from the group consisting of an infusion carrier and/or an injection carrier, preferably, the carrier is one or more carriers selected from the group consisting of physiological saline, dextrose saline, or a combination thereof.

In another preferred embodiment, the pharmaceutically acceptable carrier may be a carrier comprising nanomaterials.

In another preferred embodiment, the pharmaceutical composition is a sustained-release dosage form.

In another preferred embodiment, the dosage form of the pharmaceutical composition is lyophilized powder.

In another preferred embodiment, the lyophilized powder includes a lyophilized protectant.

In another preferred embodiment, the lyophilized protectant is selected from the group consisting of glucose, sucrose, mannitol, or a combination thereof.

In another preferred embodiment, the dosage form of the pharmaceutical composition is an inhalant.

In another preferred embodiment, the inhalant includes lyophilized powder.

In another preferred embodiment, the dosage form of the pharmaceutical composition is a liquid preparation.

In another preferred embodiment, the liquid preparation includes the polypeptide according to the first aspect of the present invention or the pharmaceutically acceptable salt thereof, an osmotic pressure regulator and water.

In another preferred embodiment, the osmotic pressure regulator includes salts and/or carbohydrates.

In another preferred embodiment, the salt includes sodium chloride.

In another preferred embodiment, the carbohydrates include glucose.

In another preferred embodiment, the dosage form of the pharmaceutical composition is a spray.

In another preferred embodiment, the dosage form of the pharmaceutical composition is an aerosol.

In another preferred embodiment, the aerosol includes the polypeptide according to the first aspect of the present invention or a pharmaceutically acceptable salt thereof and a propellant.

In another preferred embodiment, the propellant is compressed carbon dioxide, compressed nitrogen, tetrafluoroethane, heptafluoropropane, propane, n-butane, and isobutane.

In another preferred embodiment, the compressed carbon dioxide is liquid carbon dioxide.

In another preferred embodiment, the compressed nitrogen is liquid nitrogen.

In another preferred embodiment, the pharmaceutical composition can be used alone or in combination in the application of preventing, treating and/or alleviating asthma.

In another preferred embodiment, the use in combination includes: use in combination with other drugs for preventing, treating and/or relieving asthma.

In another preferred embodiment, the pharmaceutical composition further includes other drugs for preventing, treating and/or relieving asthma.

In another preferred embodiment, the other drugs for preventing, treating and/or relieving asthma are selected from the group consisting of glucocorticoids, β2 receptor agonists, anticholinergic drugs, macromolecular biological targeted drugs, or a combination thereof.

In another preferred embodiment, the glucocorticoid is selected from the group consisting of beclomethasone, fluticasone, mometasone, budesonide, ciclesonide, or a combination thereof.

In another preferred embodiment, the β2 receptor agonist includes: albuterol, terbutaline, salmeterol, formoterol, or a combination thereof.

In another preferred embodiment, the anticholinergic drug includes: ipratropium bromide, tiotropium bromide, or a combination thereof.

In another preferred embodiment, the macromolecular biological targeted drug includes: an IgE monoclonal antibody, an IL-4/IL-4R monoclonal antibody, an IL-5/IL-5R monoclonal antibody, a TSLP monoclonal antibody, or a combination thereof.

In the fourth aspect of the present invention, it provides a use of the polypeptide according to the first aspect of the present invention or a pharmaceutically acceptable salt thereof for preparing a medicament for preventing, treating and/or alleviating asthma.

In another preferred embodiment, the asthma includes: allergic asthma, non-allergic asthma, late-onset asthma, airflow-restricted asthma, obesity-type asthma, and hormone-resistant asthma.

In another preferred embodiment, the allergic asthma comprises asthma caused by an allergic agent selected from the group consisting of OVA, adjuvant aluminum hydroxide, or a combination thereof.

In another preferred embodiment, the asthma includes:
(1) asthma caused by inflammatory factors.

In another preferred embodiment, the asthma caused by inflammatory factors includes asthma caused by increased secretion or expression of inflammatory factors and/or asthma caused by enhanced activity of inflammatory factors.

In another preferred embodiment, the secretion is secretion by spleen lymphocytes, preferably, by Th2 cells.

In another preferred embodiment, the expression is selected from the group consisting of protein expression, mRNA expression, or a combination thereof.

In another preferred embodiment, the inflammatory factor includes interleukin.

In another preferred embodiment, the interleukin is selected from the group consisting of IL-4, IL-5, IL-13, or a combination thereof.

In another preferred embodiment, the prevention, treatment and/or alleviation of asthma include:
(i) inhibiting inflammatory factors;
(ii) reducing IgE levels;
(iii) reducing airway resistance;
(iv) improving lung compliance; and/or
(v) inhibiting inflammatory cell infiltration in the lung.

In another preferred embodiment, the inflammatory factor includes interleukin.

In another preferred embodiment, the interleukin is selected from the group consisting of IL-4, IL-5, IL-13, or a combination thereof.

In another preferred embodiment, inhibiting inflammatory factors includes inhibiting the secretion or expression and/or activity of inflammatory factors.

In another preferred embodiment, the IgE level is the IgE level in serum, whole blood or plasma.

In the fifth aspect of the present invention, it provides a use of the polypeptide according to the first aspect of the present invention or a pharmaceutically acceptable salt thereof for preparing a medicament for (i) inhibiting inflammation (ii) reducing IgE levels; (iii) reducing airway resistance; (iv) improving lung compliance; and/or (v) inhibiting inflammatory cell infiltration in the lungs.

In another preferred embodiment, the inflammation is caused by inflammatory factors.

In another preferred embodiment, the inflammatory factor includes interleukin.

In another preferred embodiment, the interleukin is selected from the group consisting of IL-4, IL-5, IL-13, or a combination thereof.

In the sixth aspect of the present invention, it provides a method for preventing, treating and/or alleviating asthma in mammals, comprising the steps of: administering the polypeptide according to the first aspect of the present invention or a pharmaceutically acceptable salt thereof to a subject in need.

In another preferred embodiment, the mammals include human or non-human mammals.

In another preferred embodiment, the non-human mammals include: rodents (e.g, rats, mice), primates (e.g, monkeys).

In another preferred embodiment, the asthma is selected from the group consisting of allergic asthma, non-allergic asthma, late-onset asthma, airflow-limited asthma, obesity-type asthma, hormone-resistant asthma, or a combination thereof.

In another preferred embodiment, the asthma includes: allergic asthma, non-allergic asthma, late-onset asthma, airflow-restricted asthma, obesity-type asthma, and hormone-resistant asthma.

In another preferred embodiment, the administration includes oral administration, nasal inhalation, intramuscular injection, or intravenous injection.

In the seventh aspect of the present invention, it provides a polypeptide shown in Formula II or a pharmaceutically acceptable salt thereof, wherein the polypeptide or a pharmaceutically acceptable salt thereof has activity of (a) preventing, treating and/or alleviating chronic obstructive pulmonary disease and/or (b) preventing, treating and/or alleviating asthma;

XO-X1-X2-X3-X4-X5-X6-X7-X8 (II)

wherein,
X0 and X8 are each independently none, or a peptide of 1-3 amino acids;
X1 is an amino acid selected from the group consisting of Gly, Pro, Ala;
X2 is an amino acid selected from the group consisting of Gln, Asn;
X3 is an amino acid selected from the group consisting of Thr, Ser;
X4 is an amino acid selected from the group consisting of Tyr, Trp, Phe, Thr, Ser;
X5 is an amino acid selected from the group consisting of Thr, Ser;
X6 is an amino acid selected from the group consisting of Ser, Thr;
X7 is an amino acid selected from the group consisting of Gly, Pro, Ala;
wherein, one or more amino acids of X0-X8 are each independently a glycosylation-modified amino acid.

In another preferred embodiment, the glycosylation-modified amino acid is a monoglycosylation-modified amino acid or a disaccharide-modified amino acid.

In another preferred embodiment, the monosaccharide is an L-type monosaccharide or a D-type monosaccharide

In another preferred embodiment, the monosaccharide is a pentose or a hexose.

In another preferred embodiment, the monosaccharide is a pyranose or a furanose.

In another preferred embodiment, the monosaccharide is glyceraldehyde, erythrose, threose, arabinose, ribose, xylose, lyxose, glucose, mannose, fructose or galactose.

In another preferred embodiment, the glucose group is an L-type glucose group or a D-type glucose group.

In another preferred embodiment, the disaccharide is selected from the group consisting of fructose and lactose.

In another preferred embodiment, the one or more glycosylation-modified amino acids are independently selected from the group consisting of amino acids modified by glucosyl and amino acids modified by galactosyl.

In another preferred embodiment, the glycosylation-modified amino acid is an amino acid modified by glucosyl.

In another preferred embodiment, the glycosylation-modified amino acid refers to that the sugar is connected to the amino acid through a glycosidic bond.

In another preferred embodiment, the amino acids modified by glucosyl refers to that glucose is connected to the amino acid through a glycosidic bond.

In another preferred embodiment, the glycosidic bond is -O-glycosidic bond, -N-glycosidic bond, lipoglycosidic bond or glycopeptide bond.

In another preferred embodiment, in the glycosylation-modified amino acid, the sugar is linked to the amino acid through -O-glycosidic bond, -N-glycosidic bond, lipoglycosidic bond or glycopeptide bond.

In another preferred embodiment, the amino acids in X0-X8 are each independently a D-type amino acid or an L-type amino acid.

In another preferred embodiment, the amino acids in X0-X8 are each independently a D-type amino acid.

In another preferred embodiment, 6-9, preferably 3-5, more preferably 1-2 amino acids in X0-X8 are glycosylation-modified amino acids.

In another preferred example, two of X1-X7 are glycosylation-modified amino acids, and the two glycosylation-modified amino acids are adjacent amino acids.

In another preferred embodiment, the X0 is none or a non-glycosylation-modified amino acid.

In another preferred embodiment, the X1 is a non-glycosylation-modified amino acid.

In another preferred embodiment, the X2 is a non-glycosylation-modified amino acid.

In another preferred embodiment, the X3 is a glycosylation-modified amino acid.

In another preferred embodiment, the X4 is a non-glycosylation-modified amino acid.

In another preferred embodiment, the X5 is a glycosylation-modified amino acid.

In another preferred embodiment, the X6 is a glycosylation-modified amino acid.

In another preferred embodiment, the X7 is a non-glycosylation-modified amino acid.

In another preferred embodiment, the X8 is none or a non-glycosylation-modified amino acid.

In another preferred embodiment, the polypeptide is selected from the group consisting of SIPI-G4, SIPI-G5, SIPI-G6, SIPI-G7, SIPI-G8, SIPI-G9, SIPI-G10, or a combination thereof.

In another preferred embodiment, the polypeptide is selected from the group consisting of SIPI-G5, SIPI-G9, or a combination thereof.

In another preferred embodiment, the polypeptide is selected as SIPI-G9.

In another preferred embodiment, the total length of the polypeptide is ≤20 amino acid residues, preferably ≤13 amino acid residues, preferably ≤12 amino acid residues, and preferably ≤10 amino acid residues, more preferably ≤8 amino acid residues, more preferably ≤7 amino acid residues, most preferably is 7 amino acid residues.

In another preferred embodiment, the X0 and X8 are none or peptides with 1-3 amino acids.

In another preferred embodiment, the polypeptide is selected from the following group:
(a) a polypeptide having the amino acid sequence shown in SEQ ID NO: 1, and the length of the polypeptide is 5-20 amino acids (preferably 8-12 amino acids);
(b) a derivative polypeptide derived from (a), which is formed by substitution, deletion or addition of 1-2 amino acid residues of amino acid sequence shown in SEQ ID NO: 1, and has the function of preventing, treating and/or alleviating asthma.

In another preferred embodiment, the derivative polypeptide retains ≥70%, preferably 80%, more preferably 85%, and most preferably 90% or more than 95% of activity of the polypeptide shown in SEQ ID NO: 1 for preventing, treating and/or alleviating asthma.

In another preferred embodiment, the homology between the derivative polypeptide and SEQ ID NO: 1 is ≥80%, preferably ≥90%; more preferably ≥95%.

In another preferred embodiment, the polypeptide is formed by substitution of 1-5, preferably 1-3, more preferably 1-2 amino acids; and/or
deletion of 1-3, preferably 1-2 amino acids of the polypeptide shown in SEQ ID NO.: 1; and/or
addition of 1-3, more preferably 1-2 amino acids in the two terminals of the polypetide respectively.

In another preferred embodiment, the amino acid sequence of the polypeptide is shown in SEQ ID NO: 1.

In another preferred example, 2 or 3 amino acids in the amino acid sequence shown in SEQ ID NO: 1 are glycosylation-modified amino acids.

In another preferred example, all amino acids in the amino acid sequence shown in SEQ ID NO: 1 are glycosylation-modified amino acids.

In the eighth aspect of the present invention, it provides a pharmaceutical composition, wherein the pharmaceutical composition comprising:
(a) the polypeptide according to the seventh aspect of the invention or a pharmaceutically acceptable salt thereof; and
(b) a pharmaceutically acceptable carrier or excipient.

In another preferred embodiment, the component (a) accounts for 0.1-99.9 wt %, preferably 10-99.9 wt %, more preferably 70-99.9 wt % of the total weight of the pharmaceutical composition.

In another preferred embodiment, the pharmaceutical composition is liquid, solid, or semi-solid.

In another preferred embodiment, the dosage form of the pharmaceutical composition is an oral dosage form, an injection, a spray, an aerosol or an external pharmaceutical dosage form.

In another preferred embodiment, the dosage form of the pharmaceutical composition is a subcutaneous injection or an intramuscular injection.

In another preferred embodiment, the dosage form of the pharmaceutical composition includes tablets, granules, capsules, oral liquids, or injections.

In another preferred embodiment, the pharmaceutical composition is a liquid composition.

In another preferred embodiment, the pharmaceutical composition is an oral preparation.

In another preferred embodiment, the pharmaceutically acceptable carrier is selected from the group consisting of an infusion carrier and/or an injection carrier, preferably, the carrier is one or more carriers selected from the group consisting of physiological saline, dextrose saline, or a combination thereof.

In another preferred embodiment, the pharmaceutically acceptable carrier may be a carrier comprising nanomaterials.

In another preferred embodiment, the pharmaceutical composition is a sustained-release dosage form.

In another preferred embodiment, the dosage form of the pharmaceutical composition is lyophilized powder.

In another preferred embodiment, the lyophilized powder includes a lyophilized protectant.

In another preferred embodiment, the lyophilized protectant is selected from the group consisting of glucose, sucrose, mannitol, or a combination thereof.

In another preferred embodiment, the dosage form of the pharmaceutical composition is an inhalant.

In another preferred embodiment, the inhalant includes lyophilized powder.

In another preferred embodiment, the dosage form of the pharmaceutical composition is a liquid preparation.

In another preferred embodiment, the liquid preparation includes the polypeptide according to the seventh aspect of the present invention or a pharmaceutically acceptable salt thereof, an osmotic pressure regulator and water.

In another preferred embodiment, the osmotic pressure regulator includes salts and/or carbohydrates.

In another preferred embodiment, the salt includes sodium chloride.

In another preferred embodiment, the carbohydrates include glucose.

In another preferred embodiment, the dosage form of the pharmaceutical composition is a spray.

In another preferred embodiment, the dosage form of the pharmaceutical composition is an aerosol.

In another preferred embodiment, the aerosol includes the polypeptide according to the seventh aspect of the present invention or its pharmaceutically acceptable salt and a propellant.

In another preferred embodiment, the propellant is compressed carbon dioxide, compressed nitrogen, tetrafluoroethane, heptafluoropropane, propane, n-butane, and isobutane.

In another preferred embodiment, the compressed carbon dioxide is liquid carbon dioxide.

In another preferred embodiment, the compressed nitrogen is liquid nitrogen.

In another preferred embodiment, the pharmaceutical composition can be used alone or in combination in the application of preventing, treating and/or alleviating chronic obstructive pulmonary disease.

In another preferred embodiment, the use in combination includes: use in combination with other drugs for preventing, treating and/or alleviating chronic obstructive pulmonary disease.

In another preferred embodiment, the pharmaceutical composition further includes other drugs for preventing, treating and/or alleviating chronic obstructive pulmonary disease.

In another preferred embodiment, the other drugs for preventing, treating and/or alleviating chronic obstructive pulmonary disease are selected from the group consisting of glucocorticoids, β2 receptor agonists, anticholinergic drugs, or macromolecular biological targeted drugs.

In another preferred embodiment, the glucocorticoid is selected from the group consisting of fluticasone, prednisolone, methylprednisone, or a combination thereof.

In another preferred embodiment, the β2 receptor agonist is selected from the group consisting of formoterol, olodaterol, salmeterol, or a combination thereof.

In another preferred embodiment, the anticholinergic drug is selected from the group consisting of aclidinium bromide, glycopyrronium bromide, tiotropium bromide, or a combination thereof.

In another preferred embodiment, the macromolecular biological targeted drug includes: an IgE monoclonal antibody, an IL-4/IL-4R monoclonal antibody, an IL-5/IL-5R monoclonal antibody, a TSLP monoclonal antibody, or a combination thereof.

In another preferred embodiment, the pharmaceutical composition further includes other drugs for preventing, treating and/or alleviating asthma.

In another preferred embodiment, the other drugs for preventing, treating and/or alleviating asthma are selected from the group consisting of glucocorticoids, β2 receptor agonists, anticholinergic drugs, macromolecular biological targeted drugs, or a combination thereof.

In another preferred embodiment, the glucocorticoid is selected from the group consisting of beclomethasone, fluticasone, mometasone, budesonide, ciclesonide, or a combination thereof.

In another preferred embodiment, the β2 receptor agonist includes: albuterol, terbutaline, salmeterol, formoterol, or a combination thereof.

In another preferred embodiment, the anticholinergic drug includes: ipratropium bromide, tiotropium bromide, or a combination thereof.

In another preferred embodiment, the macromolecular biological targeted drug includes: an IgE monoclonal antibody, an IL-4/IL-4R monoclonal antibody, an IL-5/IL-5R monoclonal antibody, a TSLP monoclonal antibody, or a combination thereof.

In the ninth aspect of the present invention, it provides a use of the polypeptide according to the seventh aspect of the present invention or a pharmaceutically acceptable salt thereof for preparing a medicament for (a) preventing, treatment and/or alleviating chronic obstructive pulmonary disease; and/or (b) preventing, treating and/or alleviating asthma.

In another preferred embodiment, the drug is a subcutaneous injection or an intramuscular injection.

In another preferred embodiment, the chronic obstructive pulmonary disease is selected from the group consisting of chronic obstructive bronchitis, emphysema, or a combination thereof.

In another preferred embodiment, the asthma includes: allergic asthma, non-allergic asthma, late-onset asthma, airflow-restricted asthma, obesity-type asthma, and hormone-resistant asthma.

In another preferred embodiment, the allergic asthma comprises asthma caused by an allergic agent selected from the group consisting of OVA, adjuvant aluminum hydroxide, or a combination thereof.

In another preferred embodiment, the asthma includes:
(1) asthma caused by inflammatory factors.

In another preferred embodiment, the asthma caused by inflammatory factors includes asthma caused by increased secretion or expression of inflammatory factors and/or asthma caused by enhanced activity of inflammatory factors.

In another preferred embodiment, the secretion is secretion by spleen lymphocytes, preferably, by Th2 cells.

In another preferred embodiment, the expression is selected from the group consisting of protein expression, mRNA expression, or a combination thereof.

In another preferred embodiment, the inflammatory factor includes interleukin.

In another preferred embodiment, the interleukin is selected from the group consisting of IL-4, IL-5, IL-13, or a combination thereof.

In another preferred embodiment, the prevention, treatment and/or alleviation of chronic obstructive pulmonary disease include:
(i) inhibiting inflammatory factors;
(ii) reducing IgE levels;
(iii) reducing airway resistance;
(iv) improving lung compliance; and/or
(v) inhibiting inflammatory cell infiltration in the lung.

In another preferred embodiment, the prevention, treatment and/or alleviation of asthma include:
(i) inhibiting inflammatory factors;
(ii) reducing IgE levels;
(iii) reducing airway resistance;
(iv) improving lung compliance; and/or
(v) inhibiting inflammatory cell infiltration in the lung.

In another preferred embodiment, the inflammatory factor includes interleukin.

In another preferred embodiment, the interleukin is selected from the group consisting of IL-4, IL-5, IL-13, or a combination thereof.

In another preferred embodiment, inhibiting inflammatory factors includes inhibiting the secretion or expression and/or activity of inflammatory factors.

In another preferred embodiment, the IgE level is the IgE level in serum, whole blood or plasma.

In the tenth aspect of the present invention, it provides a use of the polypeptide according to the seventh aspect of the present invention or the pharmaceutically acceptable salt thereof for preparing a medicament for (i) inhibiting inflammation; (ii) reducing IgE levels; (iii) reducing airway resistance; (iv) improving lung compliance; and/or (v) inhibiting inflammatory cell infiltration in the lung.

In another preferred embodiment, the inflammation is caused by inflammatory factors.

In another preferred embodiment, the inflammatory factor includes interleukin.

In another preferred embodiment, the interleukin is selected from the group consisting of IL-4, IL-5, IL-13, or a combination thereof.

In the eleventh aspect of the present invention, it provides a method for preventing, treating and/or alleviating chronic obstructive pulmonary disease and/or preventing, treating and/or alleviating asthma in mammals, comprising the steps of: administering the polypeptide according to the seventh aspect of the present invention or a pharmaceutically acceptable salt thereof to a subject in need.

In another preferred embodiment, the mammals include human or non-human mammals.

In another preferred embodiment, the non-human mammals include: rodents (e.g., rats, mice), primates (e.g., monkeys).

In another preferred embodiment, the chronic obstructive pulmonary disease is selected from the group consisting of chronic obstructive bronchitis, emphysema, or a combination thereof.

In another preferred embodiment, the administration includes oral administration, nasal inhalation, intramuscular injection, or intravenous injection.

It should be understood that within the scope of the present invention, each technical features of the present invention described above and in the following (such as examples) may be combined with each other to form a new or preferred technical solution, which is not listed here due to space limitations.

### DESCRIPTION OF DRAWINGS

Figure 1 shows the synthetic route of SP peptide and its derivative SIPI-D00.
Figure 2 is a graph of the blood concentration-time change curve after a single subcutaneous injection of SP (10 mg/kg) in SD rats.
Figure 3 is a graph of the blood concentration-time change curve after a single subcutaneous injection of SIPI-D00 (5 mg/kg) in SD rats.
Figure 4 shows the effects of different groups on the lung pathology of OVA-induced asthma model mice (4x).
Figure 5 shows the effects of different groups on the lung pathology of OVA-induced asthma model mice (20x).
Figure 6 shows the synthetic route of glycosylated polypeptides SIPI-G4, SIPI-G5, SIPI-G6, SIPI-G7, SIPI-G8, SIPI-G9, and SIPI-G10.
Figure 7 shows the plasma drug concentrations at different times after a single intravenous injection of SIPI-G9 (1 mg/kg) in SD rats.
Figure 8 shows the plasma drug concentrations at different times after a single subcutaneous injection of SIPI-G9 (5 mg/kg) in SD rats.
Fig. 9 shows the result of HE staining of lung pathology of smoking-induced COPD rats.
Figure 10 shows the results of Masson staining of lung pathology in smoking-induced COPD rats.
Figure 11 shows the effects of different groups on the lung pathology of OVA-induced asthma model mice (4x).
Figure 12 shows the effects of different groups on the lung pathology of OVA-induced asthma model mice (20x).

### DETAILED DESCRIPTION OF INVENTION

After extensive and intensive research, through a large number of screening, a small molecule polypeptide with a length of only 7-20 (e.g., a small peptide SIPI-D00 formed by 7 D-type amino acids, or a small peptide formed by 7 amino acids, of which 1, 2, 3, and 4 are glycosylated amino acids) which can prevent, treat and/or relieve asthma and/or chronic obstructive pulmonary function has been discovered for the first time. Specifically, in the present invention, a polypeptide molecule chemically modified at a suitable site has increased stability, prolonged action time *in vivo,* and retained biological activity. On this basis, the present inventor has completed the present invention.

### Terms

### Polypeptide

As used herein, the polypeptide of the present invention refers to the polypeptide of the structure of Formula I according to the first aspect of the present invention or a pharmaceutically acceptable salt thereof and/or the polypeptide of the structure of Formula II according to the seventh aspect of the present invention or a pharmaceutically acceptable salt thereof.

As used herein, the terms "SIPI-D00 polypeptide", "SIPI-D00 peptide", "SIPI-D00", and "D00" are used interchangeably and refer to the polypeptide having the activity of preventing, treating and/or alleviating asthma and having the structure of Formula I according to the first aspect of the present invention.

As used herein, the terms "SP polypeptide", "SP peptide" and "SP" are used interchangeably and refer to a polypeptide having the amino acid sequence shown in SEQ ID NO: 1, and from the N-terminus to the C-terminus, the amino acid sequence of the SP peptide is GQTYTSG. As used herein, the terms "D type-amino acid" and "D-amino acid" are used interchangeably. Typically, "D type-tryptophan" is used interchangeably with "D-tryptophan" and "D-Ser", and other amino acids are similar.

As used herein, the terms "SIPI-G4 polypeptide", "SIPI-G4 peptide", "SIPI-G-4" and "SP-G4" are used interchangeably, and from the N-terminus to the C-terminus, the amino acid sequence of the SIPI-G4 peptide is GQT(Glc)YT(Glc)S(Glc)G.

As used herein, the terms "SIPI-G5 polypeptide", "SIPI-G5 peptide", "SIPI-G-5" and "SP-G5" are used interchangeably, and from the N-terminus to the C-terminus, the amino acid sequence of the SIPI-G5 peptide is GQTYT(Glc)SG.

As used herein, the terms "SIPI-G6 polypeptide", "SIPI-G6 peptide", "SIPI-G-6" and "SP-G6" are used interchangeably, and from the N-terminus to the C-terminus, the amino acid sequence of the SIPI-G6 peptide is GQT(Glc)YTSG. As used herein, the terms "SIPI-G7 polypeptide", "SIPI-G7 peptide", "SIPI-G-7" and "SP-G7" are used interchangeably, and from the N-terminus to the C-terminus, the amino acid sequence of the SIPI-G7 peptide is GQTYTS(Glc)G.

As used herein, the terms "SIPI-G8 polypeptide", "SIPI-G8 peptide", "SIPI-G-8" and "SP-G8" are used interchangeably, and from the N-terminus to the C-terminus, the amino acid sequence of the SIPI-G8 peptide is GQT(Glc)YT(Glc)SG.

As used herein, the terms "SIPI-G9 polypeptide", "SIPI-G9 peptide", "SIPI-G-9" and "SP-G9" are used interchangeably, and from the N-terminus to the C-terminus, the amino acid sequence of the SIPI-G9 peptide is GQT(Glc)YTS(Glc)G.

As used herein, the terms "SIPI-G10 polypeptide", "SIPI-G10 peptide", "SIPI-G-10" and "SP-G10" are used interchangeably, and from the N-terminus to the C-terminus, the amino acid sequence of the SIPI-G10 peptide is GQTYT(Glc)S(Glc)G. As used herein, the terms "D type-amino acid" and "D-amino acid" are used interchangeably. Typically, "D type-tryptophan" is used interchangeably with "D-tryptophan" and "D-Ser", and other amino acids are similar.

As used herein, the terms "L type-amino acid" and "L-amino acid" are used interchangeably. Typically, "L type-tryptophan" is used interchangeably with "L-tryptophan" and "L-Ser", and other amino acids are similar.

In a preferred embodiment, the polypeptide of the present invention is the SP peptide derivative SIPI-D00, and the SIPI-D00 has the amino acid sequence shown in SEQ ID NO: 2, and from the N-terminus to the C-terminus, the amino acid sequence of SP peptide derivative is GSTYTQG. More preferably, all amino acid residues of the SIPI-D00 polypeptide are D-type amino acid residues.

In a more preferred embodiment, the polypeptide of the present invention has the amino acid sequence shown in SEQ ID NO: 2, and 1-2, preferably 3-5, more preferably 6-7 amino acids in the amino acid sequence shown in SEQ ID NO: 2 are D-type amino acids.

In a more preferred embodiment, the polypeptide of the present invention has the amino acid sequence shown in SEQ ID NO: 2, and all the amino acids in the amino acid sequence shown in SEQ ID NO: 2 are D-type amino acids.

As used herein, the terms "SP peptide glycosylated derivative", "glycosylated polypeptide" or "glycosylated SP peptide" are used interchangeably and refer to the glycosylated proteins or polypeptides (e.g, SIPI-G4, SIPI-G5, SIPI-G6, SIPI-G7, SIPI-G4, SIPI-G5, SIPI-G6, SIPI-G7, SIPI- G8, SIPI-G9, SIPI-G10, preferably SIPI-G5, SIPI-G9) with the amino acid sequence (SEQ ID NO: 1) which have the activity of preventing, treating and/or alleviating chronic obstructive pulmonary disease and/or preventing, treating and/or alleviating asthma.

Furthermore, the term "polypeptide" also includes variants thereof (preferably glycosylation modifications). These variant forms include (but are not limited to): deletions, insertions and/or substitution of 1-5 (usually 1-4, preferably 1-3, more preferably 1-2, most preferably 1) amino acids, and addition of one or several (usually within 5, preferably within 3, more preferably within 2) amino acids at the C-terminal and/or N-terminal. For example, in the art, substitution with amino acids of similar or similar properties generally does not alter the function of the protein. For another example, the addition of one or several amino acids at the C-terminal and/or N-terminal usually does not alter the structure and function of the protein.

The present invention also includes active fragments, derivatives and analogs of the polypeptides of the present invention. As used herein, the terms "fragments", "derivatives" and "analogs" refer to polypeptides that substantially retain the function or activity of preventing, treating and/or alleviating asthma. The polypeptide fragments, derivatives or analogs of the present invention may be (i) a polypeptide having one or more conservative or non-conservative amino acid residues (preferably conservative amino acid residues) substituted, or (ii) a polypeptide with substituted groups in one or more amino acid residues, or (iii) a polypeptide formed by fusion of the polypeptide of the present invention to another compound (such as a compound that prolongs the half-life of a polypeptide, such as polyethylene glycol), or (iv) a polypeptide formed by the fusion of an additional amino acid sequence to this polypeptide sequence (then protein is formed by fusion with a leader sequence, a secretory sequence or a tag sequence such as 6His). These fragments, derivatives and analogs are well known to those skilled in the art in light of the teachings herein.

A preferred class of active derivatives means that compared with the amino acid sequence of Formula I or Formula II, there are at most 5, preferably at most 3, more preferably at most 2, and most preferably 1 amino acid with similar or similar properties amino acids are replaced to form polypeptides. These conservative variant polypeptides are preferably formed by carrying out the amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the polypeptides of the present invention. The differences between these analogs and the polypeptides of the present invention may be differences in amino acid sequence, differences in modified forms that do not affect the sequence, or both. Analogs also include analogs with residues other than natural L-amino acids (e.g, D-amino acids), as well as analogs with non-naturally occurring or synthetic amino acids (e.g, beta, gamma-amino acids). It should be understood that the polypeptides of the present invention are not limited to the representative polypeptides exemplified above.

Modified (generally without altering the primary structure) forms include chemical derivations of polypeptides such as acetylation or carboxylation *in vivo* or *in vitro.* Modifications also include glycosylation, such as those resulting from glycosylation modifications in the synthesis and processing of the polypeptide or in further processing steps. Such modifications can be accomplished by exposing the polypeptide to enzymes that perform glycosylation, such as mammalian glycosylases or deglycosylases. Modified forms also include sequences with phosphorylated amino acid residues (e.g, phosphotyrosine, phosphoserine, phosphothreonine). Polypeptides that have been modified to improve their resistance to proteolysis or to optimize their solubility are also included.

The polypeptides of the present invention may also be used in the form of salts derived from pharmaceutically or physiologically acceptable acids or bases. These salts include, but are not limited to, salts with the following acids: hydrochloric acid, hydrobromic acid, sulfuric acid, citric acid, tartaric acid, phosphoric acid, lactic acid, pyruvic acid, acetic acid, succinic acid, oxalic acid, fumaric acid, maleic acid acid, oxaloacetic acid, methanesulfonic acid, ethanesulfonic acid, benzenesulfonic acid, or isethionic acid. Other salts include: salts formed with alkali or alkaline earth metals such as sodium, potassium, calcium or magnesium, and in the form of esters, carbamates or other conventional "prodrugs".

### Preparation method

The polypeptides of the present invention may be chemically synthesized. Accordingly, the polypeptides of the present invention can be artificially synthesized by conventional methods.

A preferred method is to use liquid phase synthesis techniques or solid phase synthesis techniques such as Boc solid phase method, Fmoc solid phase method or a combination of both methods. Solid-phase synthesis can quickly obtain samples, and an appropriate resin carrier and synthesis system can be selected according to the sequence characteristics of the target peptide. For example, the preferred solid phase carrier in the Fmoc system is Wang resin linked to the C-terminal amino acid in the peptide. The Wang resin structure is polystyrene, and the arm between the amino acid is 4-alkoxybenzyl alcohol; with 25% hexahydropyridine /Dimethylformamide was treated at room temperature for 20 minutes to remove the Fmoc protecting group and extended from the C-terminus to the N-terminus one by one according to the given amino acid sequence. After the synthesis, the synthesized proinsulin-related peptide was cleaved from the resin with trifluoroacetic acid containing 4% p-cresol and the protective group was removed. The crude peptide could be obtained by filtration to remove the resin and then ether precipitation. After the resulting product solution was lyophilized, the desired peptide was purified by gel filtration and reversed-phase high pressure liquid chromatography. When using the Boc system for solid-phase synthesis, the preferred resin is PAM resin linked to the C-terminal amino acid in the peptide. The PAM resin structure is polystyrene, and the arm between the amino acid is 4-hydroxymethyl phenylacetamide. In the Boc synthesized system, in the cycle of deprotection, neutralization and coupling, the protecting group Boc was removed with TFA/dichloromethane (DCM) and neutralized with diisopropylethylamine (DIEA)/dichloromethane. After the peptide chain condensation is completed, the peptide chain is cleaved from the resin and the protecting group is removed at the same time by treating with hydrogen fluoride (HF) containing p-cresol (5-10%) at 0 °C for 1 hour. The peptide was extracted with 50-80% acetic acid (containing a small amount of mercaptoethanol), and the solution was lyophilized and further separated and purified by molecular sieve Sephadex G10 or Tsk-40f, and then purified by high pressure liquid phase to obtain the desired peptide. Each amino acid residue can be coupled using a variety of coupling agents and coupling methods known in the art of peptide chemistry, such as diisopropyl carbonodiimide (DIC), hydroxybenzotriazole (HOBt) or 1,1,3,3-tetraurea hexafluorophosphate (HBTU) can be used for direct coupling. The purity and structure of the synthesized short peptides can be confirmed by reversed-phase high performance liquid chromatography and mass spectrometry analysis.

In a preferred embodiment, the polypeptide of the present invention is prepared by solid-phase synthesis according to its sequence, and purified by high performance liquid chromatography to obtain a high-purity lyophilized powder of the target peptide and store at -20°C.

### Chronic obstructive pulmonary disease

Chronic obstructive pulmonary disease, referred to as "COPD", is a common chronic disease with chronic bronchitis and/or emphysema characterized by airflow obstruction, which can further develop into pulmonary heart disease and respiratory failure. Usually it is caused by airway and/or alveolar abnormalities due to significant exposure to toxic particles or gases.

Typically, the chronic obstructive pulmonary disease is selected from the group consisting of chronic obstructive bronchitis, emphysema, or a combination thereof.

### Asthma

Asthma is a chronic inflammatory disease of the respiratory tract with a complex pathogenesis, which is generally believed to be caused by a combination of genetic and environmental factors. The main features of the disease are airway hypersensitivity, reversible airflow obstruction, bronchial smooth muscle spasm, and airway inflammation. Common symptoms include wheezing, dyspnea, cough, and chest tightness. In a preferred embodiment, the asthma includes:
(1) Asthma caused by inflammatory factors.

In another preferred embodiment, the asthma caused by inflammatory factors includes asthma caused by increased secretion or expression of inflammatory factors and/or asthma caused by enhanced activity of inflammatory factors.

In another preferred embodiment, the secretion is secretion by spleen lymphocytes, preferably, by Th2 cells.

In another preferred embodiment, the expression is selected from the group consisting of protein expression, mRNA expression, or a combination thereof.

In another preferred embodiment, the inflammatory factor includes interleukin.

In another preferred embodiment, the interleukin is selected from the group consisting of IL-4, IL-5, IL-13, or a combination thereof.

Typically, the asthma includes: allergic asthma, non-allergic asthma, late-onset asthma, airflow-restricted asthma, obesity-type asthma, and hormone-resistant asthma.

Typically, the asthma is allergic asthma.

In another preferred embodiment, the allergic asthma comprises asthma caused by an allergic agent selected from the group consisting of OVA, adjuvant aluminum hydroxide, or a combination thereof.

### Use

The polypeptides of the present invention can be used to prevent, treat and/or alleviate asthma and/or chronic obstructive pulmonary disease.

In the present invention, the term "prevention" refers to a method of preventing the onset of a disease and/or its attendant symptoms or protecting a subject from acquiring a disease. "Prevention" as used herein also includes delaying the onset of the disease and/or its attendant symptoms and reducing the risk of developing the disease in a subject.

"Treatment" as used in the present invention includes delaying and terminating the progression of the disease, or eliminating the disease, and does not require 100% inhibition, elimination and reversal. In some embodiments, compared to levels observed in the absence of the polypeptides of the present invention in the composition, kit, food or nutraceutical kit, combination of active ingredients, the compositions or pharmaceutical compositions of the present invention reduce, inhibit and/or reverse asthma and/or chronic obstructive pulmonary disease, e.g., by at least about 10%, at least about 30%, at least about 50%, or at least about 80%.

In a preferred embodiment, the prevention, treatment and/or alleviation of chronic obstructive pulmonary disease include:
(i) inhibiting inflammatory factors;
(ii) reducing IgE levels;
(iii) reducing airway resistance;
(iv) improving lung compliance; and/or
(v) inhibiting inflammatory cell infiltration in the lung.

In a preferred embodiment, the prevention, treatment and/or alleviation of asthma include:
(i) inhibiting inflammatory factors;
(ii) reducing IgE levels;
(iii) reducing airway resistance;
(iv) improving lung compliance; and/or
(v) inhibiting inflammatory cell infiltration in the lung.

In another preferred embodiment, the inflammatory factor includes interleukin.

In another preferred embodiment, the interleukin is selected from the group consisting of IL-4, IL-5, IL-13, or a combination thereof.

In another preferred embodiment, inhibiting inflammatory factors includes inhibiting the secretion or expression and/or activity of inflammatory factors.

In another preferred embodiment, the IgE level is the IgE level in serum, whole blood or plasma.

The present invention also provides the polypeptide of the present invention or a pharmaceutically acceptable salt thereof for use in (i) inhibiting inflammation (ii) reducing IgE levels; (iii) reducing airway resistance; (iv) improving lung compliance; and/or (v) inhibiting inflammatory cell infiltration in the lungs.

In another preferred embodiment, the inflammation is caused by inflammatory factors.

In another preferred embodiment, the inflammatory factor includes interleukin.

In another preferred embodiment, the interleukin is selected from the group consisting of IL-4, IL-5, IL-13, or a combination thereof.

### Pharmaceutical composition

The present invention also provides a pharmaceutical composition, which contains an effective amount (e.g, 0.1-99.9wt%; preferably 10-99.9wt%; more preferably, 70-99.9wt%) of the polypeptide (especially the polypeptide SIPI-D00, SIPI-G9, SIPI-G5) of the present invention, and a pharmaceutically acceptable carrier.

Generally, the polypeptides of the present invention can be formulated in a non-toxic, inert, and pharmaceutically acceptable aqueous carrier medium, usually at a pH of about 5-8, preferably about a pH of about 6-8.

As used herein, the term "effective amount" or "effective dose" refers to an amount that produces function or activity in humans and/or animals and is acceptable to humans and/or animals.

As used herein, a "pharmaceutically acceptable" ingredient is one that is suitable for use in humans and/or mammals without undue adverse side effects (e.g, toxicity, irritation, and allergy), i.e, a substance with a reasonable benefit/risk ratio. The term "pharmaceutically acceptable carrier" refers to a carrier for administration of a therapeutic agent, including various excipients and diluents.

The pharmaceutical composition of the present invention contains a safe and effective amount of the polypeptides of the present invention and a pharmaceutically acceptable carrier. Such carriers include, but are not limited to, saline, buffers, dextrose, water, glycerol, ethanol, and combinations thereof. Generally, the pharmaceutical preparation should match the mode of administration, and the pharmaceutical composition of the present invention can be prepared in the form of injection, for example, by using normal saline or an aqueous solution containing glucose and other adjuvants by conventional methods. The pharmaceutical compositions are preferably manufactured under sterile conditions. The amount of active ingredient administered is a therapeutically effective amount. The pharmaceutical preparation of the present invention can also be made into a sustained-release preparation.

In another preferred embodiment, the dosage form of the pharmaceutical composition is a spray, an injection (e.g, intramuscular or intravenous injection).

The effective amount of the protein of the present invention may vary with the mode of administration, the severity of the disease to be treated, and the like. Selection of the preferred effective amount can be determined by one of ordinary skill in the art based on various factors (e.g, through clinical trials). The factors include, but are not limited to: the pharmacokinetic parameters such as bioavailability, metabolism, half-life, etc.; the severity of the disease to be treated by the patient, the weight of the patient, the immune status of the patient, the route of administration, etc. Generally, satisfactory results are obtained when the polypeptides of the present invention are administered at a daily dose of about 0.1-1 mg/kg animal body weight (preferably 0.3-0.6 mg/kg animal body weight). For example, several divided doses may be administered daily, or the dose may be proportionally reduced, as dictated by the exigencies of the therapeutic situation.

### The main advantages of the present invention include:

(1) The polypeptide of the present invention has an effective treatment for asthma and/or chronic obstructive pulmonary disease.
(2) The polypeptide of the present invention has excellent pharmacokinetics. The subcutaneous injection of the polypeptide has excellent characteristics of absorption into the blood, the bioavailability is significantly increased, and the half-life *in vivo* is long, so that the drug effect can be exerted for a long time.
(3) The polypeptide of the present invention has the advantages of small molecular weight, low production cost, low immunogenicity, low toxicity and side effects, and good water solubility, and can be developed into aerosols.

The present invention will be further explained below in conjunction with specific embodiments. It should be understood that these embodiments are only used to illustrate the present invention and not to limit the scope of the present invention. The experimental methods without specific conditions in the following examples usually follow the conventional conditions, such as the conditions described in Sambrook et al., Molecular Cloning: Laboratory Manual (New York: Cold Spring Harbor Laboratory Press, 1989), or according to the conditions suggested by the manufacturer. Unless otherwise specified, percentages and parts are percentages by weight and parts by weight. Unless otherwise specified, the materials and reagents used in the embodiments of the present invention are all commercially available products.

### Example

### Materials and Methods

### General Method for Synthesis of SP Peptides and SP Peptide Derivatives

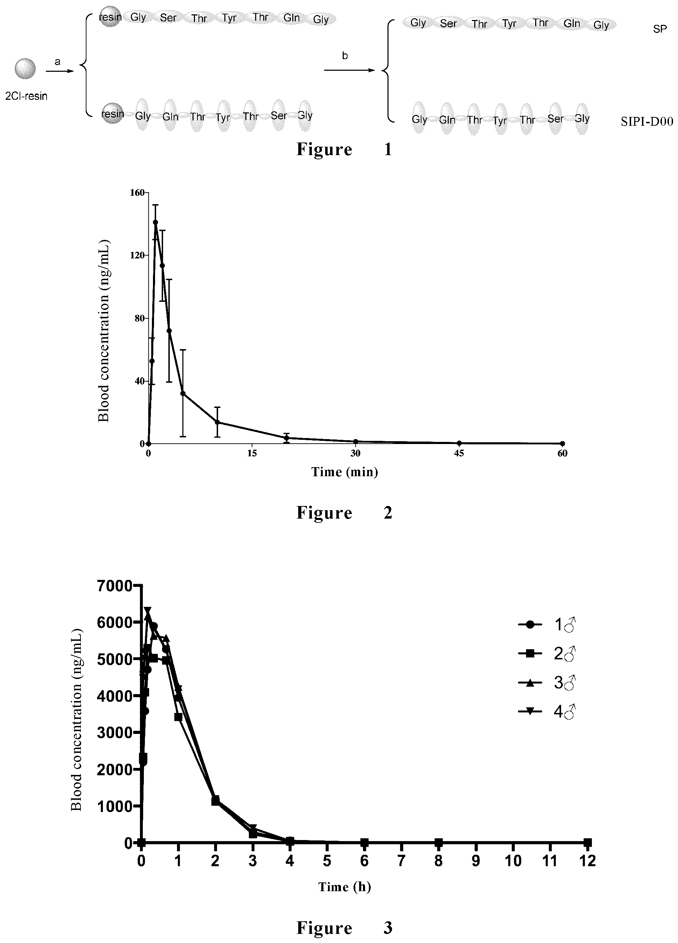

SP peptide (referred to as SP) and SP peptide derivative SIPI-D00 use the solid-phase synthesis method of the polypeptide, as shown in Figure 1. 2-Cl triphenyl resin is used as the solid-phase carrier, hydroxybenzotriazole (HOBT)/diisopropylcarbonyldiimide (DIC) is used as the condensation system, 20% piperidine in DMF solution is used as the de-Fmoc protecting group condition, the polypeptide is separated from the resin with a cleavage reagent, and the crude peptide is obtained by glacial ether precipitation. The crude peptide is purified using preparative HPLC, and pure product is identified by high-resolution mass spectrometry for molecular weight and formula matching.

### Synthesis of SP Peptide (SP) and SP Peptide Derivative D00 (SIPI-D00)

SP peptide (from N terminus to C terminus, SP peptide amino acid sequence is: GQTYTSG, amino acid sequence of SP peptide is SEQ ID NO: 1) and SIPI-D00 (from N terminus to C terminus, SIPI-D00 amino acid sequence is: GSTYTQG, amino acid sequence of SIPI-D00 peptide is SEQ ID NO: 2). The specific operation steps of synthesis are as follows:
(1) Resin swelling: 500 mg 2-Cl triphenyl resin was weighed into a peptide synthesis tube, 5-10 mL DCM was added at room temperature to fully soak the resin for at least 15 min, and then a diaphragm pump was used to remove the solvent DCM. Note: If the resin on the wall of the peptide synthesis tube cannot be completely submerged by DCM, it is necessary to use DMF to rinse the resin on the wall of the peptide synthesis tube into the bottom of the peptide synthesis tube, and then add DCM for soaking to ensure that each resin is fully swollen.
(2) Washing resin: 5-10 ml of DMF was used to rinse the wall-mounted resin into the bottom of the peptide synthesis tube, and DMF was filtered out. Then 5-10 ml DCM was added and mixed well for 1 min, and DCM was filtered off. This operation was repeated 3 times. Finally, 5-10 ml DMF was added and mixed well for 1 min, and DMF was filtered out. This operation was repeated 3 times. Note: If DMF cannot completely wash off the resin on the wall, it is necessary to be scraped into the bottom of the peptide synthesis tube with the help of external force to ensure that the reaction solution can completely soak all the resin.
(3) Condensation of the first amino acid: Fmoc-AA-OH (3 eq) was weighed and added to a 10 mL centrifuge tube, and 5 mL of DMF was taken to dissolve the amino acid, then DIPEA (8 eq) was taken and shaken in the centrifugal tube to mix well, and the amino acid was activated for one to two minutes at room temperature. The activated amino acid was poured into a peptide synthesis tube, placed in a dual-function air-bath constant temperature oscillator, shaken and reacted at 35 °C for 1 h, and washed. Note: The first amino acid of SP peptide is Fmoc-Gly-OH, and the first amino acid of SIPI-D00 is Fmoc-D-Gly-OH; the amount of DMF for dissolving the amino acid is based on whether it can completely immerse the resin, generally 3-8 mL.
(4) Washing of the polypeptide resin complex: 5-10 ml DCM was added and mixed well for 1 min, and the DCM was filtered out, and this operation was repeated 3 times. Finally, 5-10 ml DMF was added and mixed well for 1 min, and the DMF was filtered out, and this operation was repeated 3 times. Note: If DMF cannot completely wash off the resin on the wall, it needs to be scraped into the bottom of the peptide synthesis tube with the help of external force to ensure that the reaction solution can completely soak all the resin.
(5) Blocking of resin vacancies: 5 mL methanol was added to the above-mentioned polypeptide synthesis tube, placed on a dual-function air-bath constant temperature oscillator, shaken and reacted at 35°C for 20 min, and the blocking solution methanol was filtered out. This operation was repeated twice. Then the operation of washing the polypeptide resin complex in step (4) was repeated. Note: The amount of methanol in the blocking solution is based on the complete immersion of the peptide resin complex.
(6) Removal of the protecting group Fmoc: 5 mL of 20% piperidine/DMF solution was added to the above-mentioned polypeptide synthesis tube, placed in a dual-function air-bath constant temperature oscillator, shaken and reacted at 35 °C for 10 min, and the reaction solution was filtered out. This operation was repeated twice. Then the operation of washing the polypeptide resin complex in step (4) was repeated. Note: The 20% piperidine/DMF solution is based on the complete immersion of polypeptide resin complex, 20% piperidine/DMF (20 mL piperidine: 80 mL DMF).
(7) Detection of the removal of protecting Fmoc: The reaction of Fmoc protecting group removal needs to use Kaiser reagent (qualitative color development of ninhydrin) to monitor the progress of the reaction. A capillary dropper was used to take a small amount of resin into a 2 ml centrifuge tube, 3 drops of 5% ninhydrin ethanol solution and 1 drop of 80% phenol ethanol solution were added and mixed well, then heated and boiled at 200 °C for 1 min. If each resin turns blue, it means that the amino terminal is completely exposed and the Fmoc protecting group is removed completely. If a few resins are colorless, it means that the amino-terminal Fmoc protecting group has not been removed by reaction, and the above steps of removing the Fmoc protecting group need to be repeated once. Note: Kaiser reagent (5% ninhydrin ethanol solution: 80% phenol ethanol solution (3:1, v/v)); the resin used in the test should be taken from different directions as much as possible to ensure that the tested resin is representative.
(8) Condensation of the second amino acid: Fmoc-AA-OH (3 eq) and HOBT (3.6 eq) were respectively weighed into a 10 mL centrifuge tube, 6 mL DMF was added, shaken and mixd well, and then DIC (8 eq) was taken into a centrifuge tube, shaken and mixd well, and activated for one to two minutes at room temperature. The activated amino acid was added to the peptide synthesis tube, and the peptide synthesis tube was placed in a dual-function air-bath constant temperature oscillator, and reacted at 35 °C for 2 h. Then repeated the operation of washing the polypeptide resin complex in step (4). Note: The second amino acid of SP peptide is Fmoc-Ser-OH, and the second amino acid of SIPI-D00 is Fmoc-D-Gln-OH.
(9) Detection after amino acid condensation: After the amino acid condensation reaction, Kaiser reagent (qualitative color development of ninhydrin) was used to monitor the reaction process. A capillary dropper was used to take a small amount of resin into a 2 ml centrifuge tube, 3 drops of 5% ninhydrin ethanol solution and 1 drop of 80% phenol ethanol solution were added and mixed well, then heated and boiled at 200 °C for 1 min. If the resin shows colorless, the amino end is completely condensed. If it is blue or blue-violet, it indicates incomplete condensation of the amino terminal. The above-mentioned second amino acid condensation step needs to be repeated.
(10) Condensation of the remaining amino acids: the steps of (6) removal of the protecting group Fmoc, (7) detection of the removal of the protecting group Fmoc, (8) condensation of the second amino acid, and (9) detection after the amino acid condensation etc. were repeated, then the amino acids were condensed onto the resin sequentially according to the polypeptide sequence. Finally, the steps of (6) removal of protecting group Fmoc and (7) detection of the removal of the protecting group Fmoc were repeated.
(11) Separation of polypeptides and resins: After the washed polypeptide resin complex was washed with DCM for 3 times, the polypeptide resin complex were washed with diethyl ether for 3 times, and a diaphragm pump was used to dry the resin (the polypeptide resin complex is in the form of loose sand). The resin was poured into a 50 mL centrifuge tube, 20 mL cleavage reagent B reagent was weighed and added to a 50 mL centrifuge tube (containing the polypeptide resin complex), sealed at 35 °C and shaken vigorously for 4 h. After the reaction is completed, the resin and the cleavage solution (containing peptides) were filtered to separate, then the filtrate was transfered to a 50 mL centrifuge tube, and the resin was rinsed with 5 mL of B reagent. The eluent was transfered to a 50 mL centrifuge tube, and the filtrate (containing polypeptides) was concentrated by nitrogen bubbling. 40 mL glacial ether was added, and the precipitate was shaken, a centrifuge was used for centrifugation. The supernatant was poured out, and ether was added for washing. The above operations of centrifugation and ether washing were repeated three times, and the crude peptide was obtained by nitrogen blast drying. Note: the ratio of reagent B (88% TFA: 5% phenol: 5% water: 2% TIPs); the centrifugation time of the centrifuge is set to 5 min, and the rotation speed is set to 3500 r/min.
(12) Analysis, purification and identification: high performance liquid chromatograph was used to analyze the crude peptide, the crude peptide was purified with the preparation liquid, and mass spectrometer was used to identify.
   1) Analysis conditions of high performance liquid chromatograph: the deuterium lamp was turned on for dual-wavelength detection. The wavelengths are set to 214 nm and 254 nm, the flow rate is set to 1.0 mL/min; the injection volume is set to 5 µL. The gradient elution method is used for elution, and the gradient elution high performance liquid chromatograph program is set based on mobile phase B as (0 min-start, 0 min-90% mobile phase B, 2 min-90% mobile phase B, 20 min-20% mobile phase B, 30 min-0% mobile phase B, 60 min-end). Wherein mobile phase A is analytically pure acetonitrile (containing 0.1% trifluoroacetic acid), and mobile phase B is purified water (containing 0.1% trifluoroacetic acid). The analytical column is Diamonsil C18 5 µm 250 × 4.6 mM.
   2) Preparative column chromatography conditions: the deuterium lamp was turned on for dual-wavelength detection, the wavelengths are 214 nm and 254 nm, the flow rate is set to 10 mL/min; the injection volume is set to 10 mL. The gradient elution method is used for elution and purification. The gradient elution program is set based on mobile phase B as (0 min-start, 0 min-90% mobile phase B, 5 min-90% mobile phase B, 40 min- 20% mobile phase B, 60 min-0% mobile phase B, 60 min-end). The mobile phase A is analytically pure acetonitrile (containing 0.1% acetic acid), and the mobile phase B is purified water (containing 0.1% trifluoroacetic acid). The preparative column is Ryoung Tech Led C18-RPS 12 nm 10 µm 20×250 mM. The target peak solution was collected by gradient elution, the target peak solution was placed in liquid nitrogen for freezing, and then dried in a freeze dryer. The product was then analyzed by HPLC to determine the purity of the product, and mass spectrometer was used to identify whether the molecular formula and molecular weight of the product were consistent with the theoretical values.
Note: Before injection, 5% mobile phase A was used to rinse the column for more than 5 minutes. If there is no obvious impurity peak, 90% mobile phase B is used to equilibrate the column for 5-10 minutes, and an obvious equilibrium peak can be seen.

All amino acid residues of the synthetic SP peptide (SEQ ID NO: 1) are L-amino acids, and all amino acid residues of the synthetic SIPI peptide derivative SIPI-D00 (SEQ ID NO: 2) are D-amino acids.

### General Methods for Synthesis of SP Peptide Derivatives (Glycosylated SP Peptides)

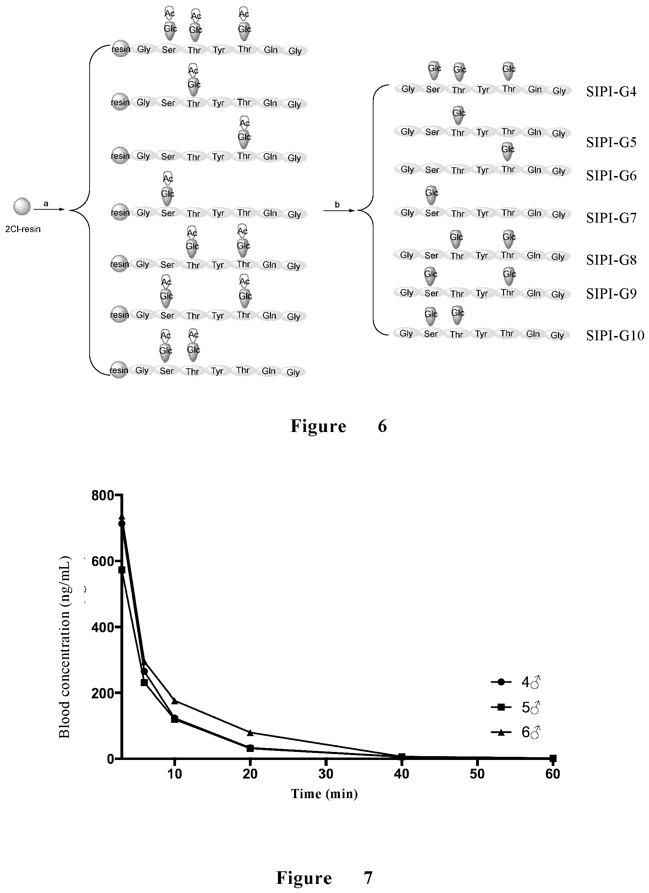

The synthesis strategy of SIPI-G4, SIPI-G5, SIPI-G6, SIPI-G7, SIPI-G8, SIPI-G9, and SIPI-G10 in SP peptide (from N-terminal to C-terminal, the amino acid sequence of SP peptide is: GQTYTSG, SEQ ID NO: 1) glycosylated derivatives is polypeptide solid-phase synthesis (as shown in Figure 6). 2-Cl triphenyl resin was used as solid support, hydroxybenzotriazole (HOBT)/diisopropyl carbonodiimide (DIC) was used as condensation system, and 20% piperidine in DMF solution was used as de-Fmoc protecting group conditions. The B cleavage reagent was used to separate the peptide from the resin, and the crude peptide was precipitated with glacial ether. The crude peptide was prepared using preparative HPLC. Purification was carried out, and the pure product was identified by molecular weight and molecular formula matching using high-resolution mass spectrometry. Finally, 5% hydrazine hydrate was used to remove the acetyl protecting group, and preparative liquid phase was used for purification. The purified glycosylated SP peptide was identified by high-resolution mass spectrometry for molecular weight and molecular formula matching.

### Synthesis of seven kinds of glycosylated polypeptides

SIPI-G4 peptide, SIPI-G5 peptide, SIPI-G6 peptide, SIPI-G7 peptide, SIPI-G8 peptide, SIPI-G9 peptide, SIPI-G10 peptide are glucose (Glc)ylated derivatives of SP peptide.

The specific operation steps for the synthesis of SIPI-G4, SIPI-G5, SIPI-G6, SIPI-G7, SIPI-G8, SIPI-G9, and SIPI-G10 are as follows:
1) Resin swelling: 500 mg 2-Cl triphenyl resin was weighed into a peptide synthesis tube, 5-10 mL DCM was added at room temperature to fully soak the resin for at least 15 min, and then a diaphragm pump was used to remove the solvent DCM. Note: If the resin on the wall of the peptide synthesis tube cannot be completely submerged by DCM, it is necessary to use DMF to rinse the resin on the wall of the peptide synthesis tube into the bottom of the peptide synthesis tube, and then add DCM for soaking to ensure that each resin is fully swollen.
2) Washing resin: 5-10 ml DMF was used to rinse the wall-mounted resin into the bottom of the peptide synthesis tube, and DMF was filtered out. Then 5-10 ml DCM was added and mixed well for 1 min, and DCM was filtered off. This operation was repeated 3 times. Finally, 5-10 ml DMF was added and mixed well for 1 min, DMF was filtered out, and this operation was repeated 3 times. Note: If DMF cannot completely wash off the resin on the wall, it is necessary to be scraped into the bottom of the peptide synthesis tube with the help of external force.
3) Condensation of the first amino acid: Fmoc-AA-OH (3 eq) was weighed and added to a 10 mL centrifuge tube, and 5 mL of DMF was taken to dissolve the amino acid, then DIPEA (8 eq) was taken and shaken in the centrifugal tube to mix well, and the amino acid was activated for one to two minutes at room temperature. The activated amino acid was poured into a peptide synthesis tube, placed in a dual-function air-bath constant temperature oscillator, shaken and reacted at 35 °C for 1 h. Note: The first amino acid is Fmoc-Gly-OH; the amount of DMF for dissolving the amino acid is based on whether it can completely immerse the resin, generally 3-8 mL.
4) Washing of the polypeptide resin complex: 5-10 ml DCM was added and mixed well for 1 min, and the DCM was filtered out, and this operation was repeated 3 times. Finally, 5-10 ml DMF was added and mixed well for 1 min, and the DMF was filtered out, and this operation was repeated 3 times. Note: If DMF cannot completely wash off the resin on the wall, it needs to be scraped into the bottom of the peptide synthesis tube with the help of external force.
5) Blocking of resin vacancies: 5 mL methanol was added to the above-mentioned polypeptide synthesis tube, placed on a dual-function air-bath constant temperature oscillator, shaken and reacted at 35°C for 20 min, and the blocking solution methanol was filtered out. This operation was repeated twice. Then the operation of washing the polypeptide resin complex in step (4) was repeated. Note: The amount of methanol in the blocking solution is based on the complete immersion of the peptide resin complex.
6) Removal of the protecting group Fmoc: 5 mL of 20% piperidine/DMF solution was added to the above-mentioned polypeptide synthesis tube, placed in a dual-function air-bath constant temperature oscillator, shaken and reacted at 35 °C for 10 min, and the reaction solution was filtered out. This operation was repeated twice. Then the operation of washing the polypeptide resin complex in step (4) was repeated. Note: The amount of 20% piperidine/DMF solution for dissolving the amino acid is based on whether it can completely immerse the resin, generally 3-8 mL.
7) Detection of the removal of the protecting group Fmoc: The reaction of protecting group Fmoc removal needs to use Kaiser reagent (qualitative color development of ninhydrin) to monitor the progress of the reaction, a capillary dropper was used to take a small amount of resin into a 2 ml centrifuge tube, 3 drops of 5% ninhydrin ethanol solution and 1 drop of 80% phenol ethanol solution were added and mixed well, then heated and boiled at 200 °C for 1 min. If each resin turns blue, it means that the amino terminal is completely exposed and the Fmoc protecting group is removed completely. If a few resins are colorless, it means that the amino-terminal Fmoc protecting group has not been removed by reaction, and the above steps of removing the Fmoc protecting group need to be repeated once. Note: Kaiser reagent (5% ninhydrin ethanol solution: 80% phenol ethanol solution (3:1, v/v)); the resin used in the test should be taken from different directions as much as possible to ensure that the tested resin is representative.
8) Condensation of the second amino acid: a) Condensation of common amino acids: Fmoc-Ser-OH (3 eq) and HOBT (3.6 eq) were weighed and added into a 10 mL centrifuge tube, 5 mL DMF was taken to dissolve amino acids and HOBT, and DIC (8 eq) was taken in a centrifuge tube, shaken and mixd well, and activated for one to two minutes at room temperature. The activated amino acids were added to the peptide synthesis tube, placed in a dual-function air-bath constant temperature oscillator, and shaken and reacted for 1 h at 35 °C. The operation of washing the polypeptide resin complex in step (4) was repeated. b) Condensation of glycosylated amino acids: Fmoc-Ser[GlcNAc(Ac4)-OH] (1.5 eq.), HOAT (1.5 eq) were weighed respectively and added to a 10 mL centrifuge tube, 4 mL DMF was added to dissolve glycosylated amino acids and HOBT. Then DIC (8 eq) was taken in a centrifuge tube, shaken and mixd well, and activated for one to two minutes at room temperature. The activated amino acids were added to the peptide synthesis tube, placed in a dual-function air-bath constant temperature oscillator, and shaken and reacted for 1 h at 35 °C. The operation of washing the polypeptide resin complex in step (4) was repeated. Note: The second amino acid of SIPI-G4, SIPI-G7, SIPI-G9 and SIPI-G10 is Fmoc-Ser[GlcNAc(Ac4)-OH], the second amino acid of SIPI-G5, SIPI-G6 and SIPI-G8 The amino acid is Fmoc-Ser-OH.
9) Detection after amino acid condensation: After the amino acid condensation reaction, Kaiser reagent (qualitative color development of ninhydrin) was used to monitor the reaction process. A capillary dropper was used to take a small amount of resin into a 2 ml centrifuge tube, 3 drops of 5% ninhydrin ethanol solution and 1 drop of 80% phenol ethanol solution were added and mixed well, then heated and boiled at 200 °C for 1 min. If the resin shows colorless, the amino end is completely condensed. If it is blue or blue-violet, it indicates incomplete condensation of the amino terminal. The above-mentioned second amino acid condensation step needs to be repeated.
10) Condensation of the remaining amino acids: the steps of (6) removal of the protecting group Fmoc, (7) detection of the removal of the protecting group Fmoc, (8) condensation of the second amino acid, and (9) detection after the amino acid condensation etc. were repeated, then the amino acids were condensed onto the resin sequentially according to the polypeptide sequence. Finally, the steps of (6) removal of protecting group Fmoc and (7) detection of the removal of the protecting group Fmoc were repeated. Note: The third amino acid of SIPI-G4, SIPI-G5, SIPI-G8 and SIPI-G10 is Fmoc-Thr[GlcNAc(Ac4)-OH], and the third amino acid of SIPI-G6, SIPI-G7 and SIPI-G9 is Fmoc-Thr-OH; the fourth amino acid of SIPI-G4, SIPI-G5, SIPI-G6, SIPI-G7, SIPI-G8, SIPI-G9, SIPI-G10 is Fmoc-Tyr-OH; the fifth amino acid of SIPI-G4, SIPI-G6, SIPI-G8 and SIPI-G9 is Fmoc-Thr[GlcNAc(Ac4)-OH], and the fifth amino acid of SIPI-G5, SIPI-G7 and SIPI-G10 is Fmoc-Thr-OH; the sixth amino acid of SIPI-G4, SIPI-G5, SIPI-G6, SIPI-G7, SIPI-G8, SIPI-G9, and SIPI-G10 is Fmoc-Gln-OH; the seventh amino acid of SIPI-G4, SIPI-G5, SIPI-G6, SIPI-G7, SIPI-G8, SIPI-G9, and SIPI-G10 is Fmoc-Gly-OH;
11) Separation of peptides and resins: After the washed polypeptide resin complex was washed with DCM for 3 times, the polypeptide resin complex were washed with diethyl ether for 3 times, and a diaphragm pump was used to dry the resin (the peptide resin complex is in the form of loose sand). The resin was poured into a 50 mL centrifuge tube, 20 mL cleavage reagent B reagent was weighed and added to a 50 mL centrifuge tube (containing the polypeptide resin complex), sealed at 35 °C and shaken vigorously for 4 h. After the reaction is completed, the resin and the cleavage solution (containing peptides) were filtered to separate, then the filtrate was transfered to a 50 mL centrifuge tube, and the resin was rinsed with 5 mL of B reagent. The eluent was transfered to a 50 mL centrifuge tube, and the filtrate (containing polypeptides) was concentrated by nitrogen bubbling. Then 40 mL glacial ether was added into the centrifuge tube, and the precipitate was shaken, a centrifuge was used for centrifugation. The supernatant was poured out, and ether was added for washing. The above operations of centrifugation and ether washing were repeated three times, and the crude peptide was obtained by nitrogen blast drying. Note: the ratio of reagent B (88% TFA: 5% phenol: 5% water: 2% TIPs); the centrifugation time of the centrifuge is set to 5 min, and the rotation speed is set to 3500 r/min.
12) Analysis, purification and identification: high performance liquid chromatograph was used to analyze the crude peptide, the crude peptide was purified with the preparation liquid, and mass spectrometer was used to identify.

Analysis conditions of high performance liquid chromatograph: the deuterium lamp was turned on for dual-wavelength detection. The wavelengths are set to 214 nm and 254 nm, the flow rate is set to 1.0 mL/min; the injection volume is set to 5 µL. The gradient elution method is used for elution, and the gradient elution high performance liquid chromatograph program is set based on mobile phase B as (0 min-start, 0 min-90% mobile phase B, 2 min-90% mobile phase B, 20 min-20% mobile phase B, 30 min-0% mobile phase B, 60 min-end). Wherein mobile phase A is analytically pure acetonitrile (containing 0.1% trifluoroacetic acid), and mobile phase B is purified water (containing 0.1% trifluoroacetic acid). The analytical column is Diamonsil C18 5 µm 250 × 4.6 mM.

Preparative column chromatography conditions: the deuterium lamp was turned on for dual-wavelength detection. The wavelengths are 214 nm and 254 nm, the flow rate is set to 10 mL/min; the injection volume is set to 10 mL. The gradient elution method is used for elution and purification. The gradient elution program is set based on mobile phase B as (0 min-start, 0 min-90% mobile phase B, 5 min-90% mobile phase B, 40 min-20% mobile phase B, 60 min-0% mobile phase B, 60 min-end). The mobile phase A is analytically pure acetonitrile (containing 0.1% acetic acid), and the mobile phase B is purified water (containing 0.1% trifluoroacetic acid). The preparative column is Ryoung Tech Led C18-RPS 12 nm 10 µm 20×250 mM. The target peak solution was collected by gradient elution, the target peak solution was placed in liquid nitrogen for freezing, and then dried in a freeze dryer. The product was then analyzed by HPLC to determine the purity of the product, and mass spectrometer was used to identify whether the molecular formula and molecular weight of the product were consistent with the theoretical values.

Note: Before injection, 5% mobile phase A was used to rinse the column for more than 5 minutes. If there is no obvious impurity peak, 90% mobile phase B is used to equilibrate the column for 5-10 minutes, and an obvious equilibrium peak can be seen.

13) Deacetylation: The purified peracetyl-protected glycopeptide was dissolved in 5% hydrazine hydrate and stirred for 2 h, and the reaction progress was monitored by HPLC.

14) Analysis, purification and identification: high performance liquid chromatograph was used to analyze the reaction solution, and the reaction solution was purified with the preparation liquid, and mass spectrometer was used to identify.

Analysis conditions of high performance liquid chromatograph: the deuterium lamp was turned on for dual-wavelength detection. The wavelengths are set to 214 nm and 254 nm, the flow rate is set to 1.0 mL/min; the injection volume is set to 5 µL. The gradient elution method is used for elution, and the gradient elution high performance liquid chromatograph program is set based on mobile phase B as (0 min-start, 0 min-99% mobile phase B, 2 min-99% mobile phase B, 20 min-50% mobile phase B, 30 min-20% mobile phase B, 60 min-end). Wherein the mobile phase A is analytically pure acetonitrile (containing 0.1% trifluoroacetic acid), and the mobile phase B is purified water (containing 0.1% trifluoroacetic acid). The analytical column is Diamonsil C18 5 µm 250 × 4.6 mM.

Preparative column chromatography conditions: the deuterium lamp was turned on for dual-wavelength detection. The wavelengths are 214 nm and 254 nm, the flow rate is set to 10 mL/min; the injection volume is set to 10 mL. The gradient elution method is used for elution and purification. The gradient elution program is set based on mobile phase B as (0 min-start, 0 min-99% mobile phase B, 5 min-99% mobile phase B, 40 min-40% mobile phase B, 60 min-0% mobile phase B, 60 min-end). The mobile phase A is analytically pure acetonitrile (containing 0.1% acetic acid), and the mobile phase B is purified water (containing 0.1% trifluoroacetic acid). The preparative column is Ryoung Tech Led C18-RPS 12 nm 10 µm 20×250 mM. The target peak solution was collected by gradient elution, the target peak solution was placed in liquid nitrogen for freezing, and then dried in a freeze dryer. The product was then analyzed by HPLC to determine the purity of the product, and mass spectrometer was used to identify whether the molecular formula and molecular weight of the product were consistent with the theoretical values.

From the N-terminus to the C-terminus, the amino acid sequence of the SIPI-G4 peptide is: GQT(Glc)YT(Glc)S(Glc)G.

From the N-terminus to the C-terminus, the amino acid sequence of the SIPI-G5 peptide is: GQTYT(Glc)SG.

From the N-terminus to the C-terminus, the amino acid sequence of the SIPI-G6 peptide is: GQT(Glc)YTSG.

From the N-terminus to the C-terminus, the amino acid sequence of the SIPI-G7 peptide is: GQTYTS(Glc)G.

From the N-terminus to the C-terminus, the amino acid sequence of the SIPI-G8 peptide is: GQT(Glc)YT(Glc)SG.

From N-terminus to C-terminus, the amino acid sequence of the SIPI-G9 peptide is: GQT(Glc)YTS(Glc)G.

From the N-terminus to the C-terminus, the amino acid sequence of the SIPI-G10 peptide is: GQTYT(Glc)S(Glc)G.

The amino acids of SP peptide, SIPI-G4, SIPI-G5, SIPI-G6, SIPI-G7, SIPI-G8, SIPI-G9, and SIPI-G10 are all L-type amino acids.

### Example 1 Stability of each 7-peptide compound in rat whole blood

### 1.1 Experimental procedure and incubation conditions

Preparation of the test product: SP and SIPI-D00 were dissolved and diluted with an appropriate amount of 50% acetonitrile water containing 0.1% formic acid to obtain a working solution with a concentration of 200 µg/mL. The usage of the stability study reagents is shown in Table 1 below.

**Table 1 Usage of Stability Study Reagents**

| Incubation | Addition volume | | Final concentration |
|---|---|---|---|
| Each compound | | 7 | 1000 ng/mL |
| Rat whole blood | | 1393 | / |

1) Fresh rat whole blood was added to the EP tube, and shaken gently to mix well;
2) The EP tube was pre-incubated in a 37 °C water bath for 5 minutes;
3) Each compound was added to start the reaction and continued to incubate at 37 °C;
4) 50 µL sample was taken from the sample tube at 0, 2 min, 5 min, 10 min, 30 min, and 60 min, respectively, into the stop tube, 50 µL of ice-cold 7% perchloric acid was added, and shaken to stop the reaction;
5) Sample was centrifuged at 13000 r/min for 10 min, 75 µL supernatant was aspirated, 60 µL 1.25% ammonia water was added and mixed well. After filtered with 0.22 µM microporous membrane, 20 µL sample was taken for LC-MS/MS analysis.

Each sample was double-piped. The results are shown in Table 2.

**Table 2 Stability of different polypeptides in rat whole blood**

| Compound | Incubation time | Recovery rate (%) M±SD |
|---|---|---|
| SP | 0 min | 100 |
| | 2 min | 64.1±7.6 |
| | 5 min | 41.3±6.8 |
| | 10 min | 28.5±0.9 |
| | 30 min | 4.93±0.7 |
| | 60 min | 1.67±0.5 |
| SIPI-D00 | 0 min | 100 |
| | 2 min | 97.9±7.4 |
| | 5 min | 101±10.7 |
| | 10 min | 99.6±1.3 |
| | 30 min | 97.9±2.5 |
| | 60 min | 103±3.9 |

It can be seen from Table 2 that the stability of SIPI-D00 peptide in whole blood is significantly higher than that of SP peptide, and the recovery rate is still as high as 100% after incubation with whole blood for 60 min, indicating that SIPI-D00 has excellent stability in whole blood. The excellent stability of SIPI-D00 makes SIPI-D00 meet the pharmacokinetic requirements of drug druggability (half-life>30min)

### Example 2 Plasma drug concentrations at different times after a single subcutaneous injection of SP peptide or SIPI-D00 peptide in SD rats

### 1. Materials

SP peptide (amino acid sequence is SEQ ID NO: 1)
SIPI-D00 polypeptide (amino acid sequence is SEQ ID NO: 2)

### 2. Experimental animals

Male SD rats with weight of about 200 g were fed in an air-conditioned constant temperature room with room temperature of 20-24°C, humidity of 40-70%, light for 12 h, and free feeding and drinking.

### 3. Experimental method

### 3.1 Method of administration

Route: Single back subcutaneous injection;
Dosage of the drug: SP peptide and SIPI-D00 peptide were prepared into 3.33 mg/mL solution with normal saline, the dosage of SP peptide by back subcutaneous injection was 10 mg/kg body weight, and the dosage of SIPI-D00 peptide by back subcutaneous injection was 5 mg/kg body weight.

### 3.2 Administration and blood sample collection

Male SD rats were fasted for 12 h before administration and had free access to water. The test substance SP peptide or SIPI-G9 polypeptide was administered by subcutaneous injection. Orbital blood was collected at different time points before and after administration. The collected whole blood was anticoagulated with 1% heparin, centrifuged at 8000 rpm for 4 min, and then 50 µL of plasma was taken into a 1.5 mL polypropylene tube and placed in a -70°C refrigerator for testing. High concentration samples were diluted with blank plasma.

### 3.3 Determination method

The plasma concentration of SIPI-G9 polypeptide was determined by high performance liquid chromatography-mass spectrometry (HPLC-MS);

### 3.3.1 HPLC-MS conditions

HPLC system: ExionLC AC system, AB Company.
MS system: Triple Quad 5500 tandem quadrupole mass spectrometer, AB Company.
Data collection: Analyst (1.7), AB Company.
HPLC chromatographic conditions:
Column: SHIM-PACK GISS C18 (2.1 × 100 mm, 1.9 µm); Injection volume: 20 µL; Mobile phase: Phase A: 0.2% acetic acid in water; Phase B: methanol, gradient elution (see Table 3 for details);

**Table 3 Gradient elution conditions**

| min | A% | B% |
|---|---|---|
| 0 | 98 | 2 |
| 0.3 | 98 | 2 |
| 0.4 | 75 | 25 |
| 2.9 | 75 | 25 |
| 3.0 | 40 | 60 |
| 4.5 | 40 | 60 |
| 4.6 | 10 | 90 |
| 5.5 | 10 | 90 |
| 5.6 | 98 | 2 |
| 7.0 | 98 | 2 |

### 3.3.2 Mass spectrometry conditions

The ion source is electrospray ionization source (ESI); the drying gas (N₂) temperature is 550 °C; the electrospray voltage is 5500V; the detection method is positive ion detection; the scanning method is the selective reaction monitoring (MRM) method, and the scanning time is 0.15 s. The mass spectrometry detection parameters are shown in Table 4 below:

**Table 4 Mass spectrometry detection parameters**

| Detection ion | Precursor ion/Da (Q 1 Mass) | Product ion/Da (Q 3 Mass) | Declustering voltage/V (DP) | Collision voltage/eV (CE) |
|---|---|---|---|---|
| SIPI-D00 | 713.3 | 305.2 | 125 | 34.5 |
| Internal standard (DX07) | 439.9 | 156.1 | 118 | 29.4 |

### 3.4 Sample processing

50 µL plasma was taken and 5 µL internal standard solution (DX07(His-Leu-Glu-Thr-Glu-Leu-His) 100 ng/mL) was added. After that, 150 µL of methanol was added to precipitate proteins, then centrifuged at 13,000 rpm for 5 min. 160 µL supernatant was taken and dried with N₂ at 40°C, then reconstituted with 75 µL of 0.1% formic acid aqueous solution. 20 µL sample was injected into HPLC-MS to determine the plasma concentration of SIPI-G9.

### 2.1.5 Data processing

Pharmacokinetic parameters were calculated using the non-compartmental model of DAS 2.0 software. Both Cₘₐₓ and Tₘₐₓ are measured values; AUC, T_{1/2}, CL and Vz are calculated by DAS 2.0.

### 4. Experimental results

After a single subcutaneous injection of SP peptide (10 mg/kg) in SD rats, the plasma drug concentrations at different times are shown in Figure 2, the pharmacokinetic parameters are shown in Table 5, and the plasma drug concentrations at different time points are shown in Table 6:

**Table 5 Main pharmacokinetic parameters after a single subcutaneous injection of SP peptide (10 mg/kg) in SD rats**

| Parameter | Unit | (mean±SD,*n*=4) |
|---|---|---|
| AUC₀₋ₜ | ng∗min/mL | 712 ± 43.0 |
| AUC_{0-∞} | ng∗min/mL | 714 ± 43.3 |
| Cₘₐₓ | ng/mL | 210 ± 21.6 |
| Tₘₐₓ | min | 1.75 ±0.957 |
| T_{1/2} | min | 8.65 ± 1.79 |

**Table 6. Plasma concentrations of SP peptide (10 mg/kg) in SD rats at different time points after a single subcutaneous injection**

| | | | | |
|---|---|---|---|---|
| Time(min) | Plasma concentration (ng/mL) | | | |
| | Parallel 1 | Parallel 2 | Parallel 3 | Parallel 4 |
| 0 | 0 | 0 | 0 | 0 |
| 0.5 | 74.16 | 40.53 | 68.78 | 39.74 |
| 1 | 98.50 | 189.92 | 230.57 | 52.68 |
| 2 | 227.39 | 54.51 | 106.46 | 100.72 |
| 3 | 93.21 | 14.50 | 56.62 | 193.41 |
| 5 | 37.95 | 11.59 | 10.66 | 93.34 |
| 10 | 15.40 | 2.46 | 6.74 | 44.67 |
| 20 | 3.16 | 1.37 | 1.03 | 7.89 |
| 30 | 1.39 | 0.46 | 0.28 | 3.07 |
| 45 | 0.31 | 0.26 | 0.14 | 0.92 |

After a single subcutaneous injection of SIPI-D00 peptide (5mg/kg) in SD rats, the plasma drug concentration at different times is shown in Figure 3, its pharmacokinetic parameters are shown in Table 7, and the plasma drug concentrations at different time points are shown in Table 8 shown:

**Table 7 Main pharmacokinetic parameters after a single subcutaneous injection of SIPI-D00 (5 mg/kg) in SD rats**

| Parameter | Unit | Mean±SD |
|---|---|---|
| AUC₀₋ₜ | ng/L^{∗}h | 8821±1316 |
| MRT₀₋ₜ | h | 0.964±0.019 |
| T_{1/2} | h | 1.50±0.36 |
| Tₘₐₓ | h | 0.209±0.083 |
| Cₘₐₓ | µg/L | 5913±447 |
| Fa | % | 96.3±13.3 |

**Table 8 Plasma concentrations of SIPI-D00 (5 mg/kg) at different time points in SD rats after a single subcutaneous injection**

| Time(h) | Plasma concentration (ng/mL) | | | |
|---|---|---|---|---|
| | Parallel 1 | Parallel 2 | Parallel 3 | Parallel 4 |
| 0 | 0 | 0 | 0 | 0 |
| 0.05 | 2199 | 2332 | 4752 | 4476 |
| 0.1 | 3584 | 4095 | 5094 | 5167 |
| 0.167 | 4715 | 5293 | 6164 | 6305 |
| 0.333 | 5889 | 5022 | 5625 | 5836 |
| 0.667 | 5258 | 4958 | 5570 | 5242 |
| 1 | 3945 | 3420 | 4248 | 4176 |
| 2 | 1160 | 1124 | 1162 | 1185 |
| 3 | 291 | 232 | 396 | 387 |
| 4 | 53.5 | 34.6 | 50.5 | 51 |
| 6 | 3.51 | 4.14 | 4.61 | 5.14 |
| 8 | 2.88 | 1.57 | 1.22 | 1.52 |
| 12 | 0.26 | 0.45 | 0.29 | 2.35 |

As can be seen from Table 5-8 and Figure 2-3, the AUC of SIPI-D00 peptide is significantly improved compared with SP, indicating that SIPI-D00 has excellent absorption into the blood and significantly increased bioavailability after subcutaneous injection, and the half-life *in vivo* is as high as 1.5h, so that the drug effect can be exerted for a long time. Therefore, it can be seen that the SIPI-D00 peptide has excellent pharmacokinetic properties.

### Example 3 The effect of SP peptide and SIPI-D00 peptide on the secretion of IL-4 from mouse splenocytes stimulated by ConA (concanavalin A)

Asthma disease is mainly an inflammatory disease mediated by Th2 cells. In the process of disease progression, Th2 cells secrete IL-4, IL-5, and IL-13. These cytokines act on eosinophils, mast cells, airway structural cells, etc., and promote the aggregation of inflammatory cells in the bronchial mucosal epithelium and airway reconstruction. Therefore, its therapeutic effect on asthma was evaluated by examining the effects of SP peptide and SIPI-D00 peptide on IL-4 secretion.

### 1. Experimental method

Three BALB/C mice were sacrificed by cervical dislocation after anesthesia. Then mice were immersed in 75% ethanol for 5 minutes. After dissection and spleen extraction, spleen lymphocytes were prepared. After cell counting, 2 × 10⁵ cells/well were added to 96-well plate, SP peptide and SIPI-D00 peptide were set as different concentration groups, and different concentrations of CsA (cyclosporine A) were set as a positive control group. Different concentrations of test substances were added to each well, and duplicate wells were set for each concentration, and the final concentration of ConA was 5 µg/mL. The plate was incubated in a 37°C 5% CO₂ incubator for 24h, the drug-containing cell suspension was aspirated from each well, then centrifuged at 200g for 15min at 4°C. The supernatant was taken, and mouse IL-4 Elisa kit was used to detect IL- 4 content.

### 3.2 Experimental results

The effects of different concentrations of SP peptide and SIPI-D00 peptide on the secretion of IL-4 by ConA-stimulated mouse splenocytes are shown in Table 9, and the inhibitory rates of IL-4 secretion are shown in Table 10:

**Table 9 Effects of different polypeptides on the secretion of IL-4 by ConA-stimulated mouse splenocytes**

| IL-4 levels of polypeptides (pg/mL) | | | IL-4 levels of CsA | |
|---|---|---|---|---|
| Peptide concentration (µg/mL) | SP | SIPI-D00 | CsA concentration (µg/mL) | Concentration (pg/mL) |
| 0 | 21.60±3.51 | 21.60±3.51 | 0 | 21.60±3.51 |
| 1 | 14.28±0.51^{∗∗} | 14.05±2.19^{∗} | 0.12 | 9.87±2.19^{∗} |
| 3 | 12.42±0.34^{∗∗} | 8.71±0.95^{∗∗} | 1.2 | 7.95±1.77^{∗} |
| 10 | 10.76±2.55^{∗} | 6.26±0.36^{∗∗} | 12 | 1.81±1.63^{∗∗} |
| 30 | 10.34±0.27^{∗∗} | 5.87±0.49^{∗∗} | 120 | 0^{∗∗} |

| | | | | |
|---|---|---|---|---|
| Note: ^{∗} means compared with the concentration of 0, p<0.05, ^{∗∗} means compared with the concentration of 0, p<0.01. | | | | |

**Table 10 Inhibitory rate of IL-4 secretion by different polypeptides on ConA-stimulated mouse splenocytes**

| Inhibitory rate of polypeptide on IL-4 secretion (%) | | | Inhibitory rate of CsA on IL-4 secretion (%) | |
|---|---|---|---|---|
| Peptide concentration (µg/mL) | SP | SIPI-D00 | CsA concentration (µg/mL) | Inhibitory rate (%) |
| 0 | | | 0 | |
| 1 | 33.90% | 34.96% | 0.12 | 54.32% |
| 3 | 42.51% | 59.68% | 1.2 | 63.20% |
| 10 | 50.21% | 71.00% | 12 | 91.64% |
| 30 | 52.15% | 72.82% | 120 | 100.0% |

As can be seen from Table 9 and Table 10, compared with SP peptide, SIPI-D00 peptide can significantly inhibit the secretion of IL-4 by ConA-stimulated mouse spleen lymphocytes, thereby it has excellent therapeutic effect on type II cytokine-predominant asthma.

### Example 4 Pharmacodynamic testing

### 1. Materials and Instruments

SIPI-D00 peptide: lyophilized powder, stored at -20°C for later use;
Positive control drug: dexamethasone sodium phosphate injection, specification: 5mg/ml, 1ml/piece; appearance and physicochemical properties: colorless liquid; storage conditions: shading, sealed, and stored in a cool place; manufacturer: Sinopharm Group Rongsheng Pharmaceutical Co., Ltd.
Albumin from chicken egg white (OVA): lot number: SLBK6445V; manufacturer: SIGMA-ALDRICH
Aluminum hydroxide adjuvant: name: Imject Alum; lot number: TJ271907A; specification: 50 mL/bottle; manufacturer: Thermo scientific.
ELISA kit: Mouse IgE ELISA Kit; lot number: GR3246691-4; manufacturer: abcam.
Atomizer: model: 403C household air compression atomizer, manufacturer: Yuyue Medical.
Animal lung function analysis system: model: AniRes2005. Manufacturer: Beijing Beilanbo Technology Co., Ltd.

### 2. Experimental animals

SPF female BALB/c mice were fed with standard sterilized chow. The drinking water of the animals was supplied from drinking bottles, and the animals were allowed to drink water freely. The rearing temperature was 20 °C ~ 22°C, the humidity was 40% ~ 70%, and the light was alternated between light and dark for 12 hours.

### 3. Experimental method

### 3.1 Animal grouping

SPF female BALB/c mice were tested in two batches, one was used to detect serum IgE and lung pathology, and the other was used to detect lung function.
The first batch: mice were divided into groups, 10 mice in each group, namely: blank control group (normal saline), model group (OVA, 20ug/mouse), SP peptide group (administered dose of 175µg/kg body weight/day, 350µg /kg body weight/day, respectively), SIPI-D00 peptide group (administered dose of 175 µg/kg body weight/day, 350 µg/kg body weight/day), and dexamethasone group (administered dose of 2 mg/kg body weight/day).
The second batch: mice were divided into groups, 10 mice in each group, namely: blank control group (normal saline), model group (OVA, dose of 20ug/mouse), SP peptide group (administered dose of 87.5µg/kg body weight/ day, 175µg/kg body weight/day, 350µg/kg body weight/day, respectively), SIPI-D00 peptide group (administered dose of 87.5µg/kg body weight/day, 175µg/kg body weight/day, 350µg/kg body weight/day), and Dexamethasone group (administered dose of 2 mg/kg body weight/day).

### 3.2 Construction and administration of allergic asthma model animals

### 3.2.1 Drug preparation and route of administration

### 3.2.1.1 Preparation and administration of OVA

### 3.2.1.1.1 OVA for Sensitization:

OVA for sensitization was dissolved in sterile PBS solution at a final concentration of 0.2mg/ mL, then an equal volume of aluminum hydroxide adjuvant was added. After shaking for 30 minutes, each mouse was intraperitoneally injected with 0.2 mL (20µg OVA per mouse).

### 3.2.1.1.2 OVA for challenge:

2% OVA solution was prepared in sterile PBS. The 2% OVA was nebulized with a nebulizer, and the mice were placed in the nebulizer inhalation box for 30 minutes per day.

### 3.2.1.1.3 Dexamethasone:

Dexamethasone sodium phosphate injection was diluted with normal saline and administered by intraperitoneal injection at a dose of 2 mg/kg.

### 3.2.1.14 Immune 7-peptide and its derivatives:

According to the dosage, SP peptide and SIPI-D00 were dissolved in normal saline respectively, and administered by subcutaneous administration on the back according to the body weight of the animals.

### 3.2.2 Construction and administration of allergic asthma model animals

Mice in the model group, SP peptide group, SIPI-D00 peptide group and dexamethasone group were sensitized by intraperitoneal injection of OVA (20µg OVA/mice) on day 0, 7, and 14, and challenged by aerosol administration of OVA (once a day for 5 consecutive days) on day 21-25 to construct asthmatic model animals. Mice in the model group were given normal saline on days 21-28; mice in different doses of SP peptide group and SIPI-D00 peptide group were injected subcutaneously with different doses of SP and SIPI-D00 on days 19-28; mice in dexamethasone group were injected intraperitoneally with 2 mg/kg/day dexamethasone on days 21-28. At the same time, 10 mice in the blank control group were fed normally from day 0 to day 28 without any treatment, and were given normal saline as a blank control.

### 4 Experimental results

### 4.1 Determination of IgE level in serum

After 28 days, blood was collected from the inner canthus of the mouse, and the serum was collected, and the IgE level in the serum was measured with an ELISA kit. The results are shown in Table 11.

**Table 11 IgE levels of blank control group, model group, SP peptide group, SIPI-D00 peptide group and dexamethasone group (n=10)**

| Group | Blank control | Model group | SP (175ug/kg) | SP (350ug/kg) | SIPI-D00 (175ug/kg) | SIPI-D00 (350ug/kg) | Dexamethasone (2mg/kg) |
|---|---|---|---|---|---|---|---|
| Serum IgE level (OD450) | 0.142±0.033 | 0.723±0.044 | 0.634 ± 0.078 | 0.595 ± 0.041 | 0.421 ± 0.044 | 0.279 ± 0.036 | 0.285 ± 0.041 |

As can be seen from Table 11, the serum IgE level in the allergic asthma mouse model group sensitized by OVA combined with adjuvant aluminum hydroxide is significantly increased (p<0.01). Compared with the model group and SP peptide group, the IgE level in the serum of the SIPI-D00 peptide treatment is significantly decreased, indicating that the SIPI-D00 peptide can significantly reduce the serum IgE level in the allergic asthma mouse model group.

### 4.2 Determination of lung function

On the 28th day, the mice were anesthetized with tracheal intubation and jugular vein intubation, and the inspiratory airway resistance and lung compliance were measured in each group of animals. Each animal was given fold increasing amounts of methacholine via the jugular vein: 0.025 mg/kg, 0.05 mg/kg, 0.1 mg/kg, 0.2 mg/kg. After each dose was given, the lung compliance curve was recorded continuously for 5 minutes, and the inspiratory airway resistance and lung compliance were calculated. The results are shown in Table 12 and Table 13.

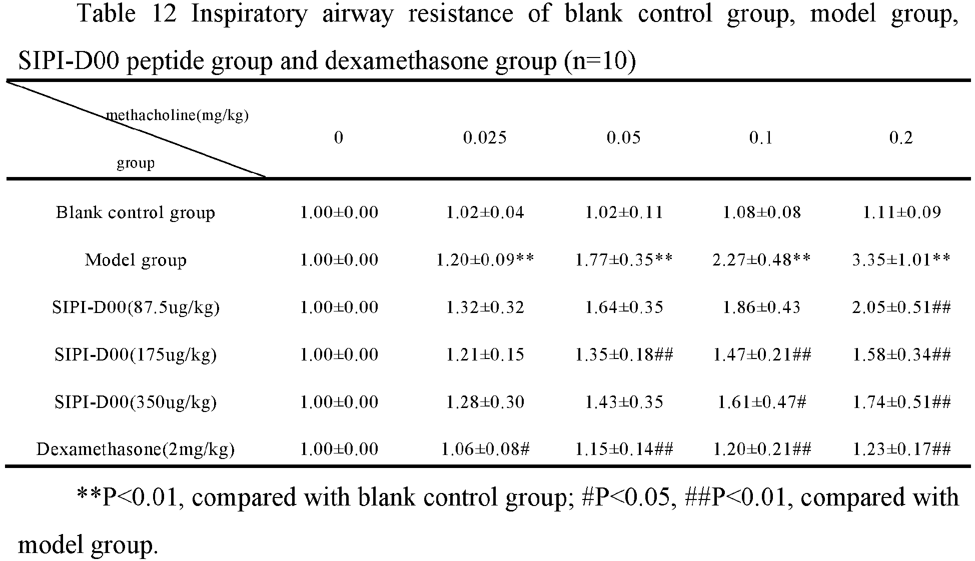

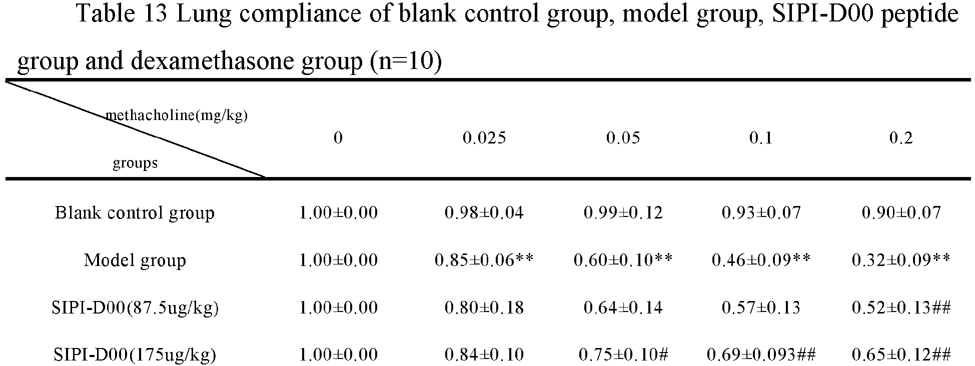

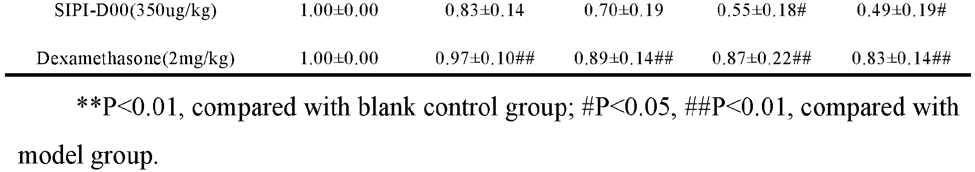

As can be seen from Table 12 and Table 13, the inspiratory phase resistance of the mice in the model group is significantly increased, and the lung compliance is significantly decreased. Compared with the model group, the inspiratory phase resistance of the mice in the dexamethasone group is significantly reduced, and the lung compliance is significantly increased; compared with the model group, the inspiratory phase resistance of the mice in the SIPI-D00 high, medium and low dose groups is significantly decreased, the lung compliance is significantly increased, indicating that the SIPI-D00 peptide can significantly reduce the inspiratory phase resistance and increase the lung compliance in mice.

### 4.3 Observation of lung pathology

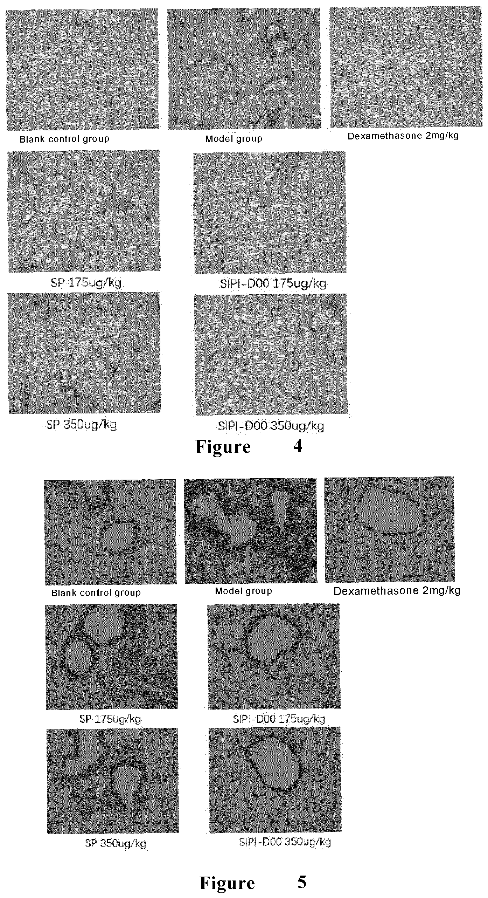

After 28 days, the mouse lung tissue was taken out, fixed in 4% formaldehyde, embedded in paraffin, sectioned, and stained with H&E for pathological observation. The results are shown in Figures 4 and 5:
Figures 4 and 5 show that there is no inflammatory cell infiltration in the lungs of the mice in the blank control group, and the alveolar cavity is clear. In the model group, a large number of inflammatory cells are infiltrated in the lungs, and the inflammatory cells are mainly eosinophils and mononuclear cells. The alveolar cavity is severely damaged, and the airway epithelium is damaged and exfoliated; the airway wall is thickened and mucosal edema; the airway exudate increases and mucus retention; the airway smooth muscle is thickened. In the positive control group, there is a small amount of inflammatory cell infiltration in the lungs, but the alveolar cavity is seriously damaged.

In SP low-dose group and high-dose group, some inflammatory cells are found in the airway mucosa; the airway wall is thickened and mucosal edema; the airway exudate increases and mucus retention; the airway smooth muscle is thickened. In SIPI-D00 low-dose group, there are more inflammatory cells infiltrating in the lungs of mice, and the inflammatory cells are mainly eosinophils and mononuclear cells, and the alveolar cavity is clear. SIPI-D00 high-dose group has less inflammatory cell infiltration and clear alveolar cavity; the airway epithelium is intact; the thickness of airway wall is normal, and there is no mucosal edema and exudation; the airway smooth muscle is normal.

It can be seen from Figure 4 and Figure 5 that the SIPI-D00 peptide can significantly reduce the infiltration of inflammatory cells in the lungs of mice.

In conclusion, SIPI-D00 peptide can effectively inhibit inflammatory factors, reduce IgE levels, reduce inspiratory airway resistance, improve lung compliance, and inhibit inflammatory cell infiltration in the lungs, thus playing an effective role in the treatment of allergic asthma.

### Example 5 Stability of each 7-peptide compound in rat whole blood

### 1.1 Experimental procedure and incubation conditions

Preparation of test products: SP, SIPI-G4, SIPI-G5, SIPI-G6, SIPI-G7, SIPI-G8, SIPI-G9, SIPI-G10 were dissolved and diluted with an appropriate amount of 50% acetonitrile water containing 0.1% formic acid to obtain working solution at a concentration of 200 µg/mL. The usage of the stability study reagents are shown in Table 14 below.

**Table 14. Usage of Stability Study Reagents**

| Incubation system | Add volume µL | | Final concentration | |
|---|---|---|---|---|
| Each compound | | 7 | | 1000 ng/mL |
| Rat whole blood | | 1393 | | / |

1) Fresh rat whole blood was added to the EP tube, and shaken gently to mix well;
2) The EP tube was pre-incubated in a 37 °C water bath for 5 minutes;
3) Each compound was added to start the reaction and continued to incubate at 37 °C;
4) 50 µL sample was taken from the sample tube at 0, 2 min, 5 min, 10 min, 30 min, and 60 min, respectively, into the stop tube, 50 µL of ice-cold 7% perchloric acid was added, and shaken to stop the reaction;
5) Sample was centrifuged at 13000 r/min for 10 min, 75 µL supernatant was aspirated, 60 µL 1.25% ammonia water was added and mixed well. After filtered with 0.22 µM microporous membrane, 20 µL sample was taken for LC-MS/MS analysis.

Each sample was double-piped, The results are shown in Table 15.

**Table 15 Stability of different peptides in rat whole blood (n=3)**

| Compound | Incubation time | Recovery rate (%) M±SD |
|---|---|---|
| SP | 0 min | 100 |
| | 2 min | 64.1±7.6 |
| | 5 min | 41.3±6.8 |
| | 10 min | 28.5±0.9 |
| | 30 min | 4.93±0.7 |
| | 60 min | 1.67±0.5 |
| SIPI-G4 | 0 min | 100 |
| | 2 min | 94.6±8.5 |
| | 5 min | 87.3±5.6 |
| | 10 min | 87.2±5.9 |
| | 30 min | 77.7±8.1 |
| | 60 min | 77.1±2.3 |
| SIPI-G5 | 0 min | 100 |
| | 2 min | 67.9±7.0 |
| | 5 min | 52.7±3.9 |
| | 10 min | 35.7±0.1 |
| | 30 min | 31.5±3.5 |
| | 60 min | 20.9±5.2 |
| SIPI-G6 | 0 min | 100 |
| | 2 min | 86.1±1.8 |
| | 5 min | 81.3±8.3 |
| | 10 min | 73.4±2.6 |
| | 30 min | 59.7±8.8 |
| | 60 min | 51.1±4.9 |
| SIPI-G7 | 0 min | 100 |
| | 2 min | 98.7±4.6 |
| | 5 min | 89.8±1.3 |
| | 10 min | 90±2.1 |
| | 30 min | 59.3±7.3 |
| | 60 min | 40.4±1.3 |
| SIPI-G8 | 0 min | 100 |
| | 2 min | 83.6±8.1 |
| | 5 min | 83.2±6.1 |
| | 10 min | 80.7±2.3 |
| | 30 min | 71.2±6.3 |
| | 60 min | 58.6±4.8 |
| SIPI-G9 | 0 min | 100 |
| | 2 min | 98.8±4.6 |
| | 5 min | 98.3±2.9 |
| | 10 min | 96.3±4.9 |
| | 30 min | 95.9±1.0 |
| | 60 min | 90.6±5.8 |
| SIPI-G10 | 0 min | 100 |
| | 2 min | 96.8±3.4 |
| | 5 min | 88.7±4.8 |
| | 10 min | 88.2±3.6 |
| | 30 min | 74.1±7.5 |
| | 60 min | 60.5±7.5 |

It can be seen from Table 15 that the stability of SIPI-G4, SIPI-G5, SIPI-G6, SIPI-G7, SIPI-G8, SIPI-G9, SIPI-G10 polypeptides in whole blood is significantly higher than that of SP peptide, especially SIPI-G9 polypeptide. After incubated with whole blood for 60min, the recovery rate is still higher than 90%, indicating that SIPI-G9 has excellent stability in whole blood, which makes it meet the pharmacokinetic requirements of compound druggability.

### Example 6 Plasma drug concentrations at different times after a single subcutaneous injection of SIPI-G9 in SD rats

### 1. Materials

SIPI-G9 polypeptide.

### 2. Experimental animals

Male SD rats with weight of about 200 g were fed in an air-conditioned constant temperature room with room temperature of 20-24°C, humidity of 40-70%, light for 12 h, and free feeding and drinking.

### 3. Experimental method

### 3.1 Method of administration

Routes: single back subcutaneous injection and single tail vein injection;
Dosage of drug: SIPI-G9 was prepared into a solution with normal saline. The dose of SIPI-G9 for subcutaneous injection on the back was 5 mg/kg body weight, and the dose for intravenous injection of SIPI-G9 was 1 mg/kg body weight.

### 3.2 Administration and blood sample collection

Male SD rats were fasted for 12 h before administration and had free access to water. The test substance SIPI-G9 polypeptide was administered by subcutaneous injection. Orbital blood was collected at different time points before and after administration. The collected whole blood was anticoagulated with 1% heparin, centrifuged at 8000 rpm for 4 min, and then 50 µL of plasma was taken into a 1.5 mL polypropylene tube and placed in a -70°C refrigerator for testing. High concentration samples were diluted with blank plasma.

### 3.3 Determination method

The plasma concentration of SIPI-G9 polypeptide was determined by high performance liquid chromatography-mass spectrometry (HPLC-MS);

### 3.3.1 HPLC-MS conditions

HPLC system: ExionLC AC system, AB Company.
MS system: Triple Quad 5500 tandem quadrupole mass spectrometer, AB Company.
Data collection: Analyst (1.7), AB Company.
HPLC chromatographic conditions:
Column: SHIM-PACK GISS C18 (2.1 × 100 mm, 1.9 µm); injection volume: 20 µL; mobile phase: phase A: 0.2% acetic acid in water; phase B: methanol, gradient elution (see Table 16 for details);

**Table 16 Gradient elution conditions**

| min | A% | B% |
|---|---|---|
| 0 | 98 | 2 |
| 0.3 | 98 | 2 |
| 0.4 | 75 | 25 |
| 2.9 | 75 | 25 |
| 3.0 | 40 | 60 |
| 4.5 | 40 | 60 |
| 4.6 | 10 | 90 |
| 5.5 | 10 | 90 |
| 5.6 | 98 | 2 |
| 7.0 | 98 | 2 |

### 3.3.2 MS conditions

The ion source is electrospray ionization source (ESI); the drying gas (N₂) temperature is 550 °C; the electrospray voltage is 5500V; the detection method is positive ion detection; the scanning method is the selective reaction monitoring (MRM) method, and the scanning time is 0.15 s. The mass spectrometry detection parameters are shown in Table 4 below:

**Table 17 Mass spectrometry detection parameters**

| Detection ion | Precursor ion /Da (Q 1 Mass) | Product ion /Da (Q 3 Mass) | Declustering voltage /V (DP) | Collision voltage /eV (CE) |
|---|---|---|---|---|
| SIPI-G9 | 560.3 | 713.5 | 89 | 22.3 |
| Internal standard (DX07) | 439.9 | 156.1 | 118 | 29.4 |

### 3.4 Sample processing

50 µL plasma was taken and 5 µL internal standard solution (DX07 (His-Leu-Glu-Thr-Glu-Leu-His) 100 ng/mL) was added. After that, 150 µL of methanol was added to precipitate proteins, then centrifuged at 13,000 rpm for 5 min, and 160 µL supernatant was taken and dried with 40°C N₂, then reconstituted with 75 µL of 0.1% formic acid aqueous solution. 20 µL sample was injected into HPLC-MS to determine the plasma concentration of SIPI-G9.

### 3.5 Data processing

Pharmacokinetic parameters were calculated using the non-compartmental model of DAS 2.0 software. Both Cₘₐₓ and Tₘₐₓ are measured values; AUC, T_{1/2}, CL and Vz are calculated by DAS 2.0.

The absolute bioavailability is calculated according to the formula Fa=(AUC₀₋ₜ)_{T}/(AUC₀₋ₜ)_{R} ×D_{R}/D_{T}×100%, wherein (AUC₀₋ₜ)_{T} is the area under the drug-time curve within 0-t of subcutaneous injection of SIPI-G9 on the back, D_{T} is the dose of subcutaneous injection of SIPI-G9 on the back (5 mg/kg body weight), (AUC₀₋ₜ)_{R} is the area under the drug-time curve within 0-t of intravenous injection of SIPI-G9, and D_{R} is the dose of intravenous injection of SIPI-G9 (1 mg/kg body weight).

### 4. Experimental results

Plasma drug concentrations of SD rats at different times after a single intravenous injection of SIPI-G9 (1 mg/kg) are shown in Table 18 and Figure 7, and the pharmacokinetic parameters are shown in Table 19:

**Table 18. Plasma drug concentrations at different times after a single intravenous injection of SIPI-G9 (1 mg/kg) in SD rats (n=3)**

| Time after treatment (min) | Plasma G-9 concentration (ng/mL) | | | | |
|---|---|---|---|---|---|
| | 4♂ | 5♂ | 6♂ | Mean | SD |
| 3 | 714 | 573 | 737 | 675 | 88.8 |
| 6 | 265 | 231 | 295 | 264 | 32.0 |
| 10 | 124 | 120 | 377 | 207 | 147 |
| 20 | 33.5 | 31.6 | 80.0 | 48.4 | 27.4 |
| 40 | 6.09 | 5.68 | 6.78 | 6.18 | 0.556 |
| 60 | 1.33 | 1.21 | 2.00 | 1.51 | 0.426 |

**Table 19 Main pharmacokinetic parameters after a single intravenous injection of SIPI-G9 (1 mg/kg) in SD rats (n=3)**

| Parameter | Unit | 4♂ | 5♂ | 6♂ | Mean | SD |
|---|---|---|---|---|---|---|
| AUC₀₋ₜ | µg/L^{∗}min | 6625 | 5487 | 8371 | 6828 | 1452 |
| MRT₀₋ₜ | min | 5.49 | 6.04 | 8.34 | 6.63 | 1.51 |
| T_{1/2} | min | 8.59 | 8.50 | 7.50 | 8.20 | 0.606 |
| Tₘₐₓ | min | 3 | 3 | 3 | 3 | 0 |
| Cₘₐₓ | µg/L | 714 | 573 | 737 | 675 | 88.8 |
| CL | L/min/kg | 0.151 | 0.182 | 0.119 | 0.151 | 0.032 |
| Vd | L/kg | 1.87 | 2.23 | 1.29 | 1.80 | 0.474 |

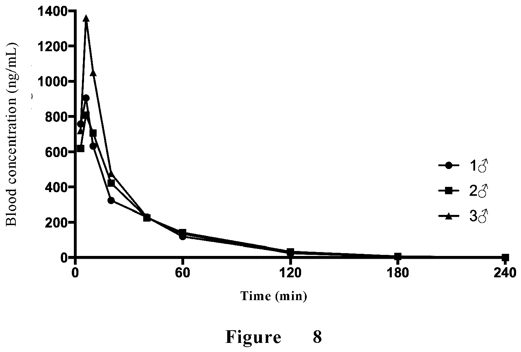

After a single subcutaneous injection of SIPI-G9 (5 mg/kg) in SD rats, the plasma drug concentrations at different times are shown in Table 20 and Figure 8, and its pharmacokinetic parameters are shown in Table 21:

**Table 20. Plasma drug concentrations at different times after a single subcutaneous injection of SIPI-G9 (5 mg/kg) in SD rats (n=3)**

| Time after treatment (min) | Plasma G-9 concentration (ng/mL) | | | | |
|---|---|---|---|---|---|
| | 1♂ | 2♂ | 3♂ | Mean | SD |
| 0 | 0 | 0 | 0 | / | / |
| 3 | 758 | 619 | 720 | 699 | 71.8 |
| 6 | 904 | 809 | 1360 | 1024 | 295 |
| 10 | 633 | 706 | 1050 | 796 | 223 |
| 20 | 324 | 423 | 478 | 408 | 78.0 |
| 40 | 230 | 226 | 229 | 228 | 2.08 |
| 60 | 118 | 141 | 134 | 131 | 11.8 |
| 120 | 28.5 | 33.5 | 24.5 | 28.8 | 4.51 |
| 180 | 6.60 | 6.15 | 5.27 | 6.01 | 0.676 |
| 240 | 0.807 | 0.763 | 0.555 | 0.708 | 0.135 |

**Table 21 Main pharmacokinetic parameters after a single subcutaneous injection of SIPI-G9 (5 mg/kg) in SD rats (n=3)**

| Parameter | Unit | 1♂ | 2♂ | 3♂ | Mean | SD |
|---|---|---|---|---|---|---|
| AUC₀₋ₜ | µg/L^{∗}min | 27049 | 28220 | 32981 | 29417 | 3142 |
| MRT₀₋ₜ | min | 34.3 | 36.8 | 30.6 | 33.9 | 3.12 |
| T_{1/2} | min | 28.8 | 26.8 | 25.7 | 27.1 | 1.59 |
| Tₘₐₓ | min | 6 | 8 | 7 | 7 | 1 |
| Cₘₐₓ | µg/L | 904 | 809 | 1360 | 1024 | 295 |
| Fa | % | 79.2 | 82.7 | 96.6 | 86.2 | 9.2 |

It can be seen from Tables 18-21 and Figures 7-8 that SIPI-G9 polypeptide has excellent pharmacokinetics whether it is injected subcutaneously or intravenously. In particular, the half-life of subcutaneous injection is as high as 27.1 min *in vivo,* indicating that it can exert effects for a long time. At the same time, compared with intravenous injection, the absolute bioavailability of subcutaneous injection is as high as 86.2%, indicating that most or almost all SIPI-G9 could enter the venous blood by subcutaneous injection. SIPI-G9 has excellent absorption into the blood by subcutaneous injection, and the absorption of SIPI-G9 by subcutaneous injection is basically similar to that of intravenous injection. Therefore, subcutaneous injection of SIPI-G9 can replace intravenous administration and has similar efficacy. Compared with intravenous administration, subcutaneous injection has the advantages of simplicity, low cost, high safety and strong patient compliance.

### Example 7 The effect of SIPI-G9 on the secretion of IL-4 from mouse splenocytes stimulated by ConA (concanavalin A)

Chronic obstructive pulmonary disease is mainly an inflammatory disease mediated by Th2 cells. In the process of disease progression, Th2 cells secrete IL-4, IL-5, and IL-13. These cytokines act on eosinophils, mast cells, airway structural cells, etc., and promote the aggregation of inflammatory cells in the bronchial mucosal epithelium and airway reconstruction. Therefore, its therapeutic effect on chronic obstructive pulmonary disease was evaluated by examining the effects of SP, SIPI-G5 and SIPI-G9 on IL-4 secretion.

### 1. Experimental method

Three BALB/C mice were sacrificed by cervical dislocation after anesthesia. Then mice were immersed in 75% ethanol for 5 minutes. After dissection and spleen extraction, spleen lymphocytes were prepared. After cell counting, 2 × 10⁵ cells/well were added to 96-well plate, SP, SIPI-G5 and SIPI-G9 were set as different concentration groups, and different concentrations of CsA (cyclosporine A) were set as a positive control group. Different concentrations of test substances were added to each well, and each concentration was set up as triple wells, and the final concentration of ConA was 5 µg/mL. The plate was incubated in a 37°C 5% CO₂ incubator for 24h, the drug-containing cell suspension was aspirated from each well, then centrifuged at 200g for 15min at 4°C. The supernatant was taken, and mouse IL-4 Elisa kit (mouse IL-4 Elisa kit) was used to detect IL- 4 content.

### 2. Experimental results

The effects of different concentrations of SP, SIPI-G5, SIPI-G9 and CsA on the secretion of IL-4 by ConA-stimulated mouse splenocytes are shown in Table 22, and the inhibitory rates of IL-4 secretion are shown in Table 23:

**Table 22 Effects of different polypeptides on the secretion of IL-4 by ConA-stimulated mouse splenocytes (n=3)**

| IL-4 level | | | | | |
|---|---|---|---|---|---|
| IL-4 levels of polypeptides (pg/mL) | | | | IL-4 levels of CsA | |
| Peptide concentration (µg/mL) | SP | SIPI-G9 | SIPI-G5 | CsA concentration (µg/mL) | concentration (pg/mL) |
| 0 | 21.60±3.51 | 21.60±3.51 | 21.60±3.51 | 0 | 21.60±3.51 |
| 1 | 14.28±0.51^{∗∗} | 15.29±0.60^{∗} | 12.48±2.69^{∗} | 0.12 | 9.87±2.19^{∗} |
| 3 | 12.42±0.34^{∗∗} | 11.44±1.12^{∗∗} | 11.26±2.74^{∗} | 1.2 | 7.95±1.77^{∗} |
| 10 | 10.76±2.55^{∗} | 8.35±1.55^{∗∗} | 12.44±2.87 | 12 | 1.81±1.63^{∗∗} |
| 30 | 10.34±0.27^{∗∗} | 7.80±2.00^{∗∗} | 12.36±2.94 | 120 | 0^{∗∗} |

| | | | | | |
|---|---|---|---|---|---|
| Note: ^{∗} means compared with the concentration of 0 p<0.05, ^{∗∗} means compared with the concentration of 0 p<0.01. | | | | | |

**Table 23 Inhibitory rate of IL-4 secretion by different polypeptides on ConA-stimulated mouse splenocytes**

| Peptide concentration (µg/mL) | Inhibitory rate on IL-4 secretion (%) | | | | |
|---|---|---|---|---|---|
| | Inhibitory rate of polypeptide on IL-4 secretion (%) | | | Inhibitory rate of CsA on IL-4 secretion (%) | |
| | SP | SIPI-G9 | SIPI-G5 | CsA concentration (µg/mL) | Inhibitory rate (%) |
| 1 | 33.90% | 29.24% | 42.24% | 0.12 | 54.32% |
| 3 | 42.51% | 47.05% | 47.86% | 1.2 | 63.20% |
| 10 | 50.21% | 61.35% | 42.42% | 12 | 91.64% |
| 30 | 52.15% | 63.88% | 42.79% | 120 | 100.0% |

It can be seen from Table 22 and Table 23 that different concentrations of SIPI-G9 can significantly inhibit the secretion of IL-4 by ConA-stimulated mouse spleen lymphocytes, thus having an excellent therapeutic effect on chronic obstructive pulmonary disease.

### Example 8 Pharmacodynamic testing 1

### 1. Materials and Instruments

SIPI-G9 peptide: lyophilized powder, stored at -20°C for later use;
Positive control drug: budesonide suspension for inhalation, specification: 2ml: 0.5mg, batch number: 1603220, properties and physicochemical properties: fine particle suspension; manufacturer: AstraZeneca Pty Ltd, Australia.
Lipopolysaccharide (LPS), batch number: 011M4001V, specification: 1mg, manufacturer: Sigma.
Atomizer: model: 403C household air compression atomizer, manufacturer: Yuyue Medical.
Animal lung function analysis system: model: AniRes2005. Manufacturer: Beijing Beilanbo Technology Co., Ltd.

### 2. Experimental animals

SPF male SD rats were fed with standard sterilized rat chow. The drinking water of the animals was supplied from drinking bottles, and the animals were allowed to drink water freely. The rearing temperature was 20 °C ∼ 22°C, the humidity was 40% ∼ 70%, and the light was alternated between light and dark for 12 hours.

### 3. Experimental method

### 3.1 Animal grouping

40 SPF male SD rats were divided into 4 groups with 10 rats in each group, namely: blank control group (normal saline), model group (smoked + LPS), SIPI-G9 group (administered dose of 350µg/kg body weight/ day), budesonide group (administered dose of 5 mg/kg body weight/day).

### 3.2 Construction and administration of chronic obstructive pulmonary disease model

Healthy male SD rats were placed in batches in a self-made organic glass airtight fumigation box (85cm×85cm×45cm), 6 days of a week, smoked for 40min a day, divided into 2 batches, with a rest of 10min in between. Each batch was given 24 cigarettes with a smoke dose of 400-500ppm. 200 µg LPS (1 g/L solution prepared with normal saline) was instilled into the airway once every 2 weeks. This cycle continued for 12 weeks to construct model animals.

From the 13th week, the constructed model animals were divided into model group, SIPI-G9 group and budesonide group, with 10 rats in each group. Model group rats continued to be modeled according to the above methods, SIPI-G9 group continued to be modeled according to the above methods with given by SIPI-G9 (aerosol administration, once a day for a total of 4 weeks), and the budesonide group continued to be modeled according to the above methods with given by budesonide (aerosol administration, once a day and for a total of 4 weeks), and ended after week 16.

At the same time, 10 rats in the blank control group were given a normal diet from 0 days to 16 weeks without any treatment, and were given normal saline as a blank control.

### 3.3 Determination of lung function

After the experiment, the rats were anesthetized with tracheal intubation and jugular vein intubation, and airway resistance and lung compliance were measured in each group of animals. Each animal was given fold increasing amounts of methacholine via the jugular vein: 0.025 mg/kg, 0.05 mg/kg, 0.1 mg/kg, 0.2 mg/kg. The lung compliance curve was recorded continuously for 5 minutes after each dose was administered. The pulmonary ventilation and ventilation percentage of rats in different groups are shown in Table 24 and Table 25.

**Table 24 The effect of SIPI-G9 on 200ms pulmonary ventilation in COPD model rats**

| Group | 200ms pulmonary ventilation (mL) |
|---|---|
| Blank control group | 79.20±1.79 |
| Model group | 67.41±4.20^{∗∗} |
| SIPI-G9 group | 84.98±3.71## |
| Budesonide group | 77.17±11.20## |

| | |
|---|---|
| ^{∗∗} compared with blank control group, P<0.01, ## compared with model group, P<0.01. | |

**Table 25 The effect of SIPI-G9 on the percentage of 200ms pulmonary ventilation in COPD model rats**

| Group | Percentage of 200ms pulmonary ventilation (%) |
|---|---|
| Blank control group | 475.4±13.1 |
| Model group | 399.8±25.4^{∗∗} |
| SIPI-G9 group | 528.6±32.6## |
| Budesonide group | 465.8±77.8## |

| | |
|---|---|
| ^{∗∗} compared with blank control group, P<0.01, ## compared with model group, P<0.01. | |

It can be seen from Table 24 and Table 25 that the 200ms pulmonary ventilation and the percentage of pulmonary ventilation of the rats in the model group are significantly reduced. Compared with the model group, the 200ms pulmonary ventilation and the percentage of pulmonary ventilation in the budesonide group are significantly increased; the 200ms pulmonary ventilation and the percentage of pulmonary ventilation in the SIPI-G9 group are significantly increased. It shows that the SIPI-G9 treatment group significantly improves and decreases the inspiratory phase resistance of the rats, increases the pulmonary ventilation and the percentage of pulmonary ventilation, and increases the lung compliance.

### 3.4 Observation of lung pathology

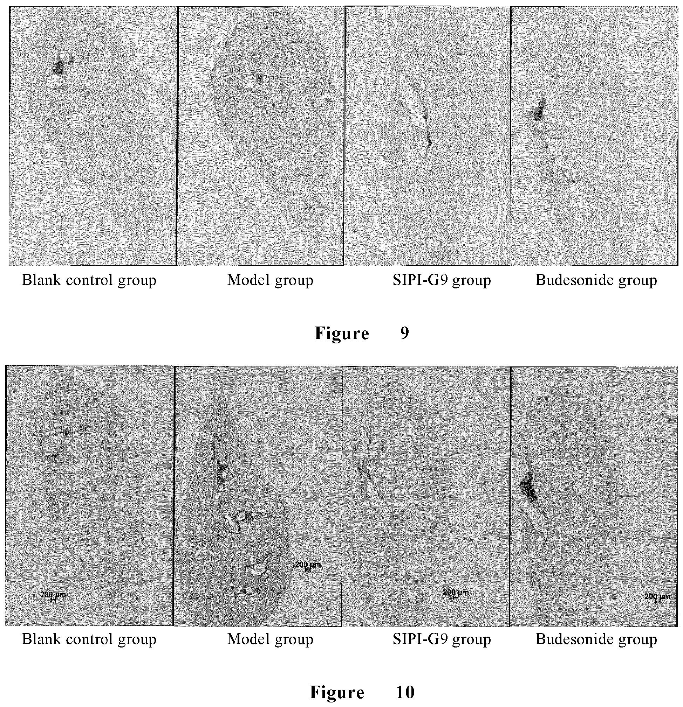

After 16 weeks, the rat lung tissue was taken out, fixed in 4% formaldehyde, embedded in paraffin, sectioned, stained with H&E and Masson for pathological observation, and the staining results are shown in Figure 9 and Figure 10.

It can be seen from Figure 9 that there is no inflammatory cell infiltration in the lungs of the rats in the blank control group, and the alveolar cavity is clear. In the model group, a large number of inflammatory cells are infiltrated in the lungs, and the inflammatory cells are mainly eosinophils and mononuclear cells, and the alveolar cavity is severely damaged. In the budesonide group, there is a small amount of inflammatory cell infiltration in the lungs, but the alveolar cavity is severely damaged. In the SIPI-G9 group, there is less inflammatory cell infiltration in the lungs, and the alveolar cavity is clear.

It can be seen from Figure 10 that there is almost no collagen deposition in the lungs of the rats in the blank control group, a large amount of collagen deposition in the lungs of the rats in the model group, a small amount of collagen deposition in the lungs of the rats in the budesonide group, and almost no collagen deposition in the lungs of the rats in the SIPI-G9 group.

In summary, SIPI-G9 can effectively reduce the inspiratory phase resistance of rats, increase pulmonary ventilation and the percentage of pulmonary ventilation, increase lung compliance, reduce pulmonary inflammatory cell infiltration and collagen deposition, thus playing an effective role in the treatment of chronic obstructive pulmonary disease models.

### Example 9 Pharmacodynamic testing 2

### 1. Materials and Instruments

SIPI-G5 peptide and SIPI-G9 peptide: lyophilized powder, stored at -20°C for later use;
Positive control drug: dexamethasone sodium phosphate injection, specification: 5mg/ml, 1ml/piece; appearance and physicochemical properties: colorless liquid; storage conditions: shading, sealed, and stored in a cool place; manufacturer: Sinopharm Group Rongsheng Pharmaceutical Co., Ltd.
Albumin from chicken egg white (OVA): lot number: SLBK6445V; manufacturer: SIGMA-ALDRICH
Aluminum hydroxide adjuvant: name: Imject Alum; lot number: TJ271907A; specification: 50 mL/bottle; manufacturer: Thermo scientific.
ELISA kit: Mouse IgE ELISA Kit; lot number: GR3246691-4; manufacturer: abcam.
Atomizer: model: 403C household air compression atomizer, manufacturer: Yuyue Medical.
Animal lung function analysis system: model: AniRes2005. Manufacturer: Beijing Beilanbo Technology Co., Ltd.

### 2. Experimental animals

SPF female BALB/c mice were fed with standard sterilized chow. The drinking water of the animals was supplied from drinking bottles, and the animals were allowed to drink water freely. The rearing temperature was 20 °C ∼ 22°C, the humidity was 40% ∼ 70%, and the light was alternated between light and dark for 12 hours.

### 3. Experimental method

### 3.1 Animal grouping

SPF female BALB/c mice were tested in two batches, one was used to detect serum IgE and lung pathology, and the other was used to detect lung function.

The first batch: mice were divided into groups, 10 mice in each group, namely: blank control group (normal saline), model group (OVA, 20ug/mice), and SP peptide group (administered dose of 175µg/kg body weight/day, 350µg /kg body weight/day, respectively), SIPI-G5 peptide group (administered dose of 175 µg/kg body weight/day, 350 µg/kg body weight/day), dexamethasone group (administered dose of 2 mg/kg body weight/day).

The second batch: mice were divided into groups, 10 mice in each group, namely: blank control group (normal saline), model group (OVA, dose of 20ug/mice), SP peptide group (administered dose of 87.5µg/kg body weight/ day, 175µg/kg body weight/day, 350µg/kg body weight/day, respectively), G9 peptide group (administered dose of 87.5µg/kg body weight/day, 175µg/kg body weight/day, 350µg/kg body weight/day), dexamethasone group (administered dose of 2 mg/kg body weight/day).

### 3.2 Construction and administration of allergic asthma model animals

### 3.2.1 Drug preparation and route of administration

### 3.2.1.1 Preparation and administration of OVA

### 3.2.1.1.1 OVA for Sensitization:

OVA for sensitization was dissolved in sterile PBS solution at a final concentration of 0.2mg/ mL, then an equal volume of aluminum hydroxide adjuvant was added. After shaking for 30 minutes, each mouse was intraperitoneally injected with 0.2 mL (20µg OVA per mouse).

### 3.2.1.1.2 OVA for challenge:

2% OVA solution was prepared in sterile PBS. The 2% OVA was nebulized with a nebulizer, and the mice were placed in the nebulizer inhalation box for 30 minutes a day.

### 3.2.1.1.3 Dexamethasone:

Dexamethasone sodium phosphate injection was diluted with normal saline and administered by intraperitoneal injection at a dose of 2 mg/kg.

### 3.2.1.14 Immune 7-peptide and its derivatives:

According to the dosage, SP peptide, SIPI-G5 peptide and SIPI-G9 were dissolved in normal saline respectively, and administered by subcutaneous administration on the back according to the body weight of the animals.

### 3.2.2 Construction and administration of allergic asthma model animals

Mice in the model group, SP peptide group, SIPI-G5 peptide group, SIPI-G9 peptide group and dexamethasone group were sensitized by intraperitoneal injection of OVA (20µg OVA/mice) on day 0, 7, and 14, and challenged by aerosol administration of OVA (once a day for 5 consecutive days) on day 21-25 to construct asthmatic model animals. Mice in the model group were given normal saline on days 21-28; mice in different doses of SP peptide group, SIPI-G5 peptide group, SIPI-G9 peptide group were injected subcutaneously with different doses of SP peptide, SIPI-G5 peptide and SIPI-G9 peptide on days 19-28; mice in dexamethasone group were injected intraperitoneally with 2 mg/kg/day dexamethasone on days 21-28. At the same time, 10 mice in the blank control group were fed normally from day 0 to day 28 without any treatment, and were given normal saline as a blank control.

### 4 Experimental results

### 4.1 Determination of IgE level in serum

After 28 days, blood was collected from the inner canthus of the mouse, and the serum was collected, and the IgE level in the serum was measured with an ELISA kit. The results are shown in Table 26.

**Table 26 IgE levels of blank control group, model group, SP peptide group, SIPI-G5 peptide group, SIPI-G9 peptide group and dexamethasone group (n=10)**

| | Blank control | Model group | SP (175ug/kg) group | SP (350ug/kg) group | SIPI-G5 (175ug/kg) group | SIPI-G5 (350ug/kg) group | SIPI-G9 (175ug/kg) group | SIPI-G9 (350ug/kg) group | Dexamethasone (2mg/kg) |
|---|---|---|---|---|---|---|---|---|---|
| Serum IgE level (OD450) | 0.142±0.033 | 0.723±0.044 | 0.634 ± 0.078 | 0.595 ± 0.041 | 0.638 ± 0.073 | 0.640 ± 0.045 | 0.404 ± 0.052 | 0.341 ± 0.030 | 0.285 ± 0.041 |

As can be seen from Table 26, the serum IgE level in the allergic asthma mouse model group sensitized by OVA combined with adjuvant aluminum hydroxide is significantly increased (p<0.01). Compared with the model group and SP peptide group, the IgE level in the serum of the SIPI -G9 peptide treatment is significantly decreased, indicating that the SIPI-G9 peptide can significantly reduce the serum IgE level in the allergic asthma mouse model group.

### 4.2 Determination of lung function

On the 28th day, the mice were anesthetized with tracheal intubation and jugular vein intubation, and the inspiratory airway resistance and lung compliance were measured in each group of animals. Each animal was given fold increasing amounts of methacholine via the jugular vein: 0.025 mg/kg, 0.05 mg/kg, 0.1 mg/kg, 0.2 mg/kg. After each dose was given, the lung compliance curve was recorded continuously for 5 minutes, and the inspiratory airway resistance and lung compliance were calculated. The results are shown in Table 27 and Table 28.

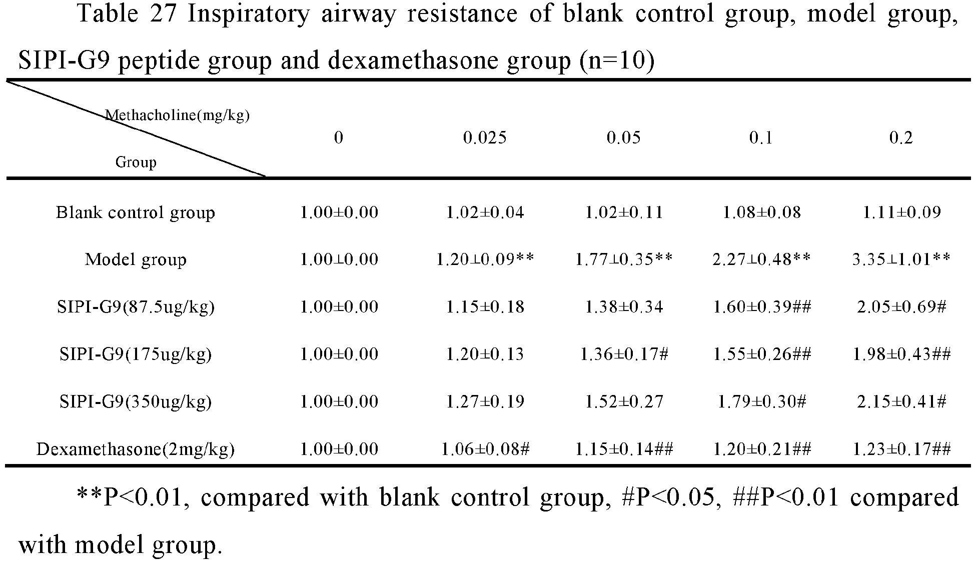

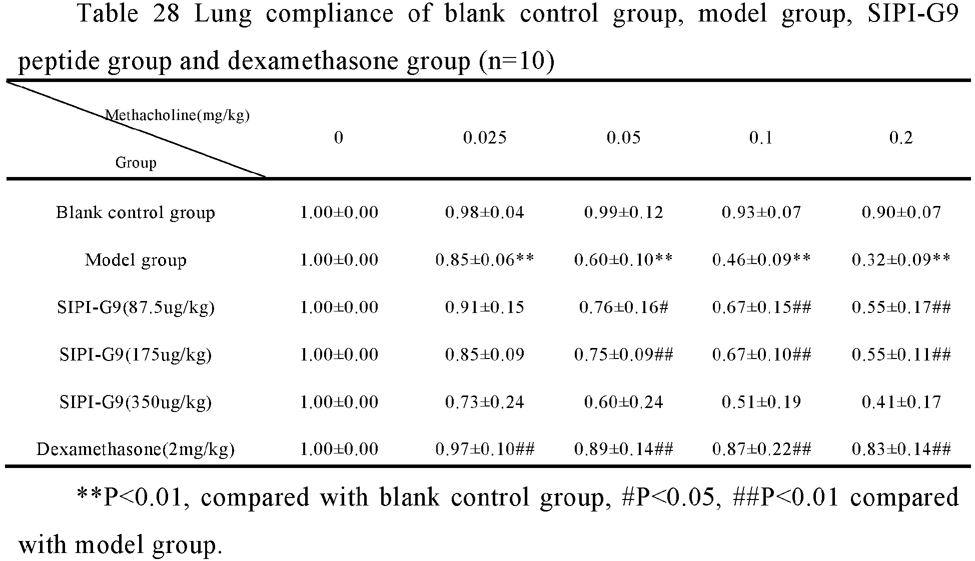

As can be seen from Table 27 and Table 28, the inspiratory phase resistance of the mice in the model group is significantly increased, and the lung compliance is significantly decreased. Compared with the model group, the inspiratory phase resistance of the mice in the dexamethasone group is significantly reduced, and the lung compliance is significantly increased; compared with the model group, the inspiratory phase resistance of the mice in the SIPI-G9 high, medium and low dose groups is significantly decreased, the lung compliance is significantly increased, indicating that the SIPI-G9 peptide can significantly reduce the inspiratory phase resistance and increase the lung compliance in mice.

### 4.3 Observation of Lung pathology

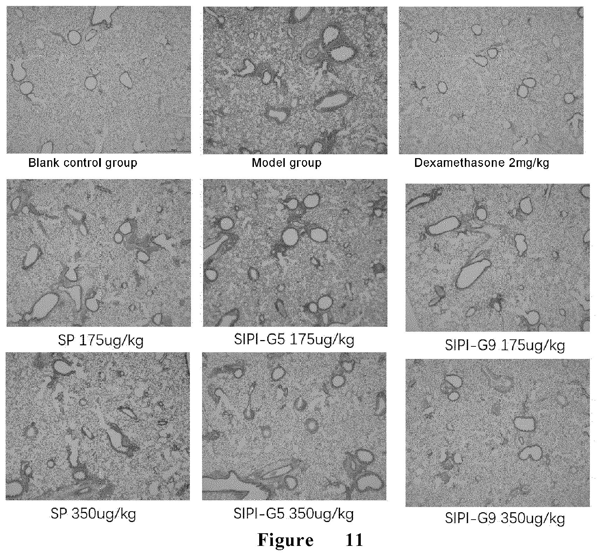

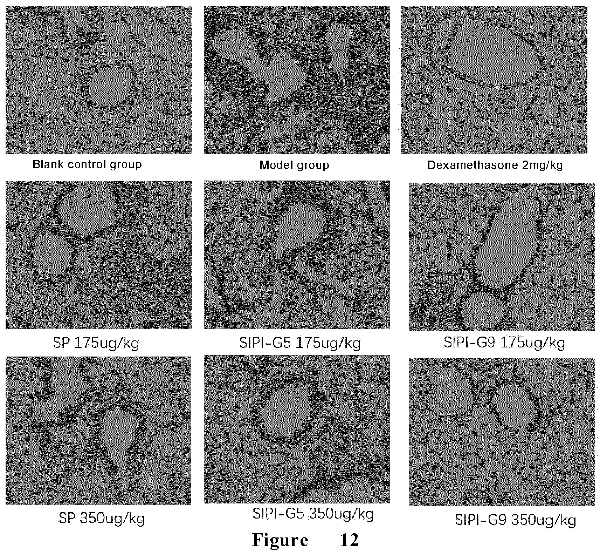

After 28 days, the mouse lung tissue was taken out, fixed in 4% formaldehyde, embedded in paraffin, sectioned, and stained with H&E for pathological observation. The results are shown in Figures 11 and 12:
It can be seen from Figure 11 and Figure 12 that there is no inflammatory cell infiltration in the lungs of the mice in the blank control group, and the alveolar cavity is clear. In the model group, a large number of inflammatory cells are infiltrated in the lungs, and the inflammatory cells are mainly eosinophils and mononuclear cells. The alveolar cavity is severely damaged, and the airway epithelium is damaged and exfoliated; the airway wall is thickened and mucosal edema; the airway exudate increased and mucus retention; the airway smooth muscle is thickened. In the positive control group, there is a small amount of inflammatory cell infiltration in the lungs, but the alveolar cavity is seriously damaged.

In SP low-dose group and high-dose group, some inflammatory cells are found in the airway mucosa; the airway wall is thickened and mucosal edema; the airway exudate increased and mucus retention; the airway smooth muscle is thickened.

In SIPI-G5 low-dose group, there are more inflammatory cells infiltrating in the lungs of mice, and some inflammatory cells are observed in the airway mucosa of mice in the high-dose group. In both low-dose and high-dose groups, it is observed that the airway wall is thickened and mucosal edema; the airway exudate increased and mucus retention; and the airway smooth muscle is thickened.

At the same dose as the SIPI-G5 group, there are less inflammatory cell infiltration in the lungs of the mice in the SIPI-G9 group, and the alveolar cavity is clear; the airway epithelium is relatively intact; the airway wall thickness is normal, and there is no mucosal edema and exudation; the smooth muscle of the tract is normal.

It can be seen from Figure 11 and Figure 12 that the SIPI-G9 peptide can significantly reduce the infiltration of inflammatory cells in the lungs of mice.

In conclusion, it can be seen that SIPI-G9 peptide can effectively inhibit inflammatory factors, reduce IgE levels, reduce inspiratory airway resistance, improve lung compliance and inhibit inflammatory cell infiltration in the lungs, so it plays an effective role in the treatment of allergic asthma.

All literatures mentioned in the present application are incorporated by reference herein, as though individually incorporated by reference. In addition, it should be understood that after reading the above teaching content of the present invention, various changes or modifications may be made by those skilled in the art, and these equivalents also fall within the scope as defined by the appended claims of the present application.

## Claims

1. A polypeptide as shown in Formula I or a pharmaceutically acceptable salt thereof, the polypeptide or a pharmaceutically acceptable salt thereof has the activity of preventing, treating and/or alleviating asthma;
X0-X1-X2-X3-X4-X5-X6-X7-X8 (I)
wherein,
X0 and X8 are each independently none, or a peptide of 1-3 amino acids;
X1 is an amino acid selected from the group consisting of Gly, Pro, Ala;
X2 is an amino acid selected from the group consisting of Ser, Thr;
X3 is an amino acid selected from the group consisting of Thr, Ser;
X4 is an amino acid selected from the group consisting of Tyr, Trp, Phe, Thr, Ser;
X5 is an amino acid selected from the group consisting of Thr, Ser;
X6 is an amino acid selected from the group consisting of Gln, Asn;
X7 is an amino acid selected from the group consisting of Gly, Pro, Ala.

2. The polypeptide of claim 1, wherein the polypeptide is substituted by 1-5, preferably 1-3, more preferably 1-2 amino acids from the polypeptide shown in SEQ ID NO.: 2; and/or
after 1-3, preferably 1-2 amino acid deletions; and/or
the two ends of the polypeptide are respectively formed by adding 1-3, more preferably 1-2 amino acids.

3. The polypeptide of claim 1, wherein the amino acid sequence of the polypeptide is shown in SEQ ID NO: 2.

4. An isolated nucleic acid molecule, wherein the nucleic acid molecule encodes the polypeptide of claim 1.

5. A pharmaceutical composition, wherein the pharmaceutical composition comprising:
(a) the polypeptide of claim 1 or a pharmaceutically acceptable salt thereof; and
(b) a pharmaceutically acceptable carrier or excipient.

6. A use of the polypeptide of claim 1 or a pharmaceutically acceptable salt thereof for preparing a medicament for preventing, treating and/or alleviating asthma.

7. The use of claim 6, wherein the asthma is selected from the group consisting of allergic asthma, non-allergic asthma, late-onset asthma, airflow-restricted asthma, obesity-type asthma, hormone-resistant asthma, or a combination thereof.

8. A polypeptide of Formula II or a pharmaceutically acceptable salt thereof, wherein the polypeptide or a pharmaceutically acceptable salt thereof has activity of (a) preventing, treating and/or alleviating chronic obstructive pulmonary disease and/or (b) preventing, treating and/or alleviating asthma;
X0-X1-X2-X3-X4-X5-X6-X7-X8 (II)
wherein,
X0 and X8 are each independently none, or a peptide of 1-3 amino acids;
X1 is an amino acid selected from the group consisting of Gly, Pro, Ala;
X2 is an amino acid selected from the group consisting of Gln, Asn;
X3 is an amino acid selected from the group consisting of Thr, Ser;
X4 is an amino acid selected from the group consisting of Tyr, Trp, Phe, Thr, Ser;
X5 is an amino acid selected from the group consisting of Thr, Ser;
X6 is an amino acid selected from the group consisting of Ser, Thr;
X7 is an amino acid selected from the group consisting of Gly, Pro, Ala;
wherein, one or more amino acids of X0-X8 are each independently a glycosylation-modified amino acid.

9. The polypeptide of claim 8, wherein the polypeptide is selected from the group consisting of SIPI-G4, SIPI-G5, SIPI-G6, SIPI-G7, SIPI-G8, SIPI-G9, SIPI-G10, or a combination thereof.

10. The polypeptide of claim 8, wherein the glycosylation-modified amino acid is an amino acid modified by glucosyl.

11. The polypeptide of claim 8, wherein the amino acid sequence of the polypeptide is shown in SEQ ID NO: 1.

12. A pharmaceutical composition, wherein the pharmaceutical composition comprising:
(a) the polypeptide of claim 8 or a pharmaceutically acceptable salt thereof; and
(b) a pharmaceutically acceptable carrier or excipient.

13. A use of the polypeptide of claim 8 or a pharmaceutically acceptable salt thereof for preparing a medicament for (a) preventing, treatment and/or alleviating chronic obstructive pulmonary disease; and/or (b) preventing, treating and/or alleviating asthma.

14. The use of claim 13, wherein the chronic obstructive pulmonary disease is selected from the group consisting of chronic obstructive bronchitis, emphysema, or a combination thereof; and/or
the asthma includes: allergic asthma, non-allergic asthma, late-onset asthma, airflow-restricted asthma, obesity-type asthma, and hormone-resistant asthma.

15. A use of the polypeptide of claim 1 or a pharmaceutically acceptable salt thereof or the polypeptide as shown in Formula II of claim 8 or a pharmaceutically acceptable salt thereof for preparing a medicament for (i) inhibiting inflammation; (ii) reducing IgE levels; (iii) reducing airway resistance; (iv) improving lung compliance; and/or (v) inhibiting inflammatory cell infiltration in the lung.
